(19) Europäisches Patentamt
European Patent Office
Office européen des brevets

(11) EP 2 383 573 A1

(12) EUROPEAN PATENT APPLICATION
published in accordance with Art. 153(4) EPC

(43) Date of publication:
**02.11.2011 Bulletin 2011/44**

(21) Application number: **10735787.3**

(22) Date of filing: **26.01.2010**

(51) Int Cl.:
***G01N 33/50*** (2006.01) ***A61K 45/00*** (2006.01)
***A61P 31/00*** (2006.01) ***G01N 33/15*** (2006.01)

(86) International application number:
**PCT/JP2010/050937**

(87) International publication number:
**WO 2010/087319 (05.08.2010 Gazette 2010/31)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **27.01.2009 JP 2009015118**

(71) Applicant: **Astellas Pharma Inc.**
**Tokyo 103-8411 (JP)**

(72) Inventors:
- **FURUTANI, Takashi**
  **Tokyo 103-8411 (JP)**
- **ENJO, Kentaro**
  **Tokyo 103-8411 (JP)**
- **KIKUCHI, Aya**
  **Tokyo 103-8411 (JP)**
- **KUROMITSU, Sadao**
  **Tokyo 103-8411 (JP)**
- **IDEYAMA, Yukitaka**
  **Tokyo 103-8411 (JP)**
- **SUZUKI, Tomoyuki**
  **Tokyo 103-8411 (JP)**
- **KURIHARA, Ryoko**
  **Tokyo 103-8411 (JP)**

(74) Representative: **Bates, Philip Ian**
**Reddie & Grose**
**16 Theobalds Road**
**London**
**WC1X 8PL (GB)**

# (54) SCREENING METHOD FOR SUBSTANCE USEFUL AS AGENT FOR TREATING PROSTATE CANCER

(57) [Object] A screening method for a compound which is useful as an agent for treating 17βHSD type 5-related diseases and/or an agent for treating 17βHSD type 5-related cancer such as prostate cancer is provided.

[Means for Solution] The present invention has been completed by establishing a screening method for a compound which is useful for treating 17βHSD type 5-related diseases, by manifesting a tumor by transplanting tumor cells to, for example, an immunodeficient mouse, topically administering a steroid which is a biosynthetic substance for a hormone into the tumor, and measuring the level of a hormone produced in the tumor.

EP 2 383 573 A1

## Description

Technical Field

The present invention relates to a screening method for a substance which is useful as an agent for treating 17βHSD type 5-related diseases and/or 17βHSD type 5-related cancer such as prostate cancer.

Background Art

Benign Prostatic Hyperplasia (BPH) is a disease mainly occurring in elder males aged 50 years or above and accompanying urinary disorders, and its incidence rate increases with the age. The number of patients with BPH in Japan has been constantly increasing in recent years with the rapid aging of the population. BPH remarkably deteriorates the quality of life of the aged males due to urinary disorders, and it is an important disease in terms of medical economics since it is the most frequently diagnosed and treated disease in the medical field of urology.

It has been found that two factors, that is, direct urethral compression due to hypertrophy of the prostate (mechanical obstruction) and elevation of intraurethral pressure due to overcontraction of the prostatic smooth muscle via the sympathetic nerve (functional obstruction), are simultaneously involved in urinary disorders accompanying BPH. Drug therapy can deal with both of these mechanisms, and 5α-reductase inhibitors are mainly used for the mechanical obstruction and α1-sympatholytic agents (α1 blockers) are mainly used for the functional obstruction. 5α reductase inhibitors regress the prostate due to their anti-androgenie effect based on the suppression of the conversion of testosterone to 5α-dehydrotestosterone (DHT) which is a more potent androgen produced by a 5α-reductase, Only the prostatic epithelium is regressed, however, and it takes a long period of time (several weeks to several months) for the drug efficacy to become apparent. On the other hand, since α1-blockers exert their drug efficacy swiftly after administration and are excellent in safety, α1-blockers are now the first-line agent for treating BPH. However, as a result of the long-term clinical studies, since a 5α-reductase inhibitor significantly delayed the transfer to invasive therapy as compared with the single use of an α1-blocker, and the like ("The New England Journal of Medicine", 2003, Vol. 349, p. 2387-2398), the usefulness of 5α-reductase inhibitors has recently been recognized again.

It has been considered that DHT in the prostate is produced by 5α-reductase from testosterone, which is produced in the testes and secreted endocrinologically to the prostate. It has been reported recently, however, that about half of DHT and its precursor, testosterone, in prostate, are synthesized from dehydroepiandrosterone (DHEA), a steroid derived from an adrenal, in cells of the prostate ("Frontier in Neuroendocrinology", 2001, Vol. 22, p. 185-212). This kind of sex hormone production system in the cells of the sex hormone target organs is called intracrinology.

It is difficult for 5a-reductase inhibitors to inhibit the local testosterone synthesis (intracrine testosterone synthesis) in the prostate. For example, it has been reported that the concentration of DHT in the prostate of the patients with BPH decreased after the administration of finasteride, a 5α-reductase inhibitor, to about 20% of the concentration before the administration, while the concentration of testosterone, a precursor, in the prostate inversely increased 4-fold ("The Journal of Urology", 1999, Vol. 161, p. 332-337). It means that although the 5α-reductase inhibitor has an effect of suppressing DHT concentration in the prostate, it has no effect of suppressing the concentration of testosterone in the prostate and instead elevates the concentration. Since testosterone has an androgen receptor binding activity of about the half of that of DHT, this local elevation of the concentrations of testosterone in the prostate is considered to be partly responsible for insufficient drug efficacy of finasteride for BPH.

Anti-androgen therapies using surgical castration and gonadotropin releasing hormone agonists are also used in prostate cancer. These anti-androgen therapies have been reported to exert an insufficient effect of suppressing the concentrations of testosterone in the prostate. For example, in patients with prostate cancer who receive the anti-androgen therapy above, the concentration of testosterone in the blood decreased to about 10% of the concentration before the therapy, while the concentration of DHT in the prostate remained at about 50% ("The Journal of Clinical Endocrinology and Metabolism", 1995, Vol. 80, p. 1066-1071). It suggests that the concentration of testosterone in the prostate is also not sufficiently decreased. Further, androgen receptors were localized also in nuclei of prostate cancer recurring after anti-androgen therapy (Hormone Refractory Prostate Cancer), and no significant difference was observed between the concentration of testosterone in recurrent prostate cancer tissues and that in the normal prostate ("Clinical Cancer Research", 2004, Vol. 10, p. 440-448). These reports strongly suggest that the effect of decreasing the concentrations of testosterone in the prostate in existing therapeutic methods is quite insufficient for treating recurrent prostate cancer and that suppression of the testosterone synthesizing mechanism in the prostate, that is, intracrine testosterone synthesis in the prostate may be a new target of prostate cancer therapy

Based on the known arts described above, since inhibitors of intracrine testosterone synthesis in the prostate have an effect of decreasing the concentrations of testosterone in the prostate and no effect of decreasing the concentrations of testosterone in the blood, the inhibitors are expected to be very attractive agent for treating BPH and/or an agent for treating prostate cancer, (1) which can decrease not only the concentration of testosterone but also the

concentration of DHT in the prostate and (2) which can avoid the adverse effects due to the suppression of the concentration of the testosterone derived from testes in the blood.

17β-hydroxysteroid dehydrogenase (17βHSD) is essential for the biosynthesis of testosterone. There are several subtypes of 17βHSD. 17βHSD type 5 is highly expressed in a human prostate and increases of the expression were reported for prostate cancer and recurrent prostate cancer ("Steroids", 2004, Vol. 69, p. 795-801; and "Cancer Research", 2006, Vol. 66, p. 2815-2825). On the other hand, almost all the testosterone in the blood is produced by 17βHSD type 3 in testes and the expression of 17βHSD type 3 is rarely observed in other tissues including the prostate ("Nature Genetic", 1994, Vol. 7, p. 34-39). 17βHSD type 5 is thus considered to be responsible for the intracrine testosterone synthesis in the prostate and selective inhibitors for 17βHSD type 5 are expected to suppress intracrine testosterone synthesis in the prostate selectively. Further, since the contribution of 17βHSD type 5 has been pointed out also in estrogen-dependent tissues such as the mammary gland and the like, the selective inhibitors are expected to be effective for estrogen-dependent diseases such as breast cancer and the like ("Endocrine Reviews", 2003, Vol. 24, p. 152-182). In addition, since AKR1C3 (another name for 17βHSD type 5), which is a subtype of aldo-keto reductase (AKR), metabolizes Polycyclic Aromatic Hydrocarbon (PAH) to generate reactive oxygen, species (ROS) ("The Journal of Biological Chemistry", 2002, Vol. 277, No. 27, p. 24799-24808) and since single nucleotide polymorphism (SNP) of AKR1C3 gene relating to oxidative stress correlates with a risk of lung cancer ("Carcinogenesis", 2004, Vol. 25, No. 11, p. 2177-2181), it is suggested that the activity of AKR1C3 in the lungs increases the risk of lung cancer via generation of ROS from PAH. Thus, selective inhibitors of 17βHSD type 5 are expected to be effective for lung cancer.

As 17βHSD type 5 inhibitors, steroid derivatives (Patent Document 1) and NSAIDs (Non-steroidal Anti-Inflammatory Drugs) such as flufenamic acid, Indomethacin and the like (Non-Patent Document 1), cinnamic acid derivatives (Non-Patent Document 2) and the like have been reported. Although the mechanism of action is different, a certain type of indazole derivatives is known to be effective for BPH (Patent Document 2).

Meanwhile, a certain type of 3-(indol-1-y1)benzoic acid derivatives (Patent Document 3), 3-(1H-benzimidazol-1-yl)benzoic acid derivatives having a heat shock protein (HSP90)-inhibitory activity (Patent Document 4), or 3-(1H-benzimidazol-l-yl)benzoic acid derivatives having a telomerase-inhibitory activity (Patent Document 5) is known to be effective for prostatic diseases such as prostate cancer. Further, a certain type of 3-(indol-1-yl)benzoic acid derivatives (Patent Document 6), or a certain type of 3-(1H-benzimidazol-1-yl)benzoic acid derivatives (Patent Documents 7 to 12) such as 3-[5-(trifluoromethyl)-1H-benzimidazol-1-yl]benzoic acid is known to be effective for central nervous system disorders such as Alzheimer's disease and dementia. Further, other 3-(1H-benzimidazol-1-yl)berrzoic acid derivatives are also known to be effective for arteriosclerosis (Patent Document 13). In addition, 3-(1H-benzimidazol-1-yl)benxoic acid derivatives having an antiviral activity (Patent Document 14), a vascular endothelial growth factor (VEGF) receptor-antagonistic activity (Patent Document 15), or a platelet-derived growth factor (PDGF)-inhibitory activity (Non-Patent Documents 3 to 5), and 3-(indol-1-yl)benzoic acid derivatives (Patent Document 16) effective for pain or the like, are known. However, there is no report about the compounds effective for BPH, prostate cancer, or the like, as described in the present application.

In order to produce an agent for treating 17βHSD type 5-related diseases such as prostate cancer or breast cancer, it is indispensable to perform a screening using a model animal by which multiple test substances can be efficiently evaluated. However, there have been almost no reports on a model animal in which such clinical conditions are reflected.

List of the Documents

Patent Document

[Patent Document 1] Pamphlet of International Publication WO 99/046279
[Patent Document 2] Pamphlet of International Publication WO 2004/064735
[Patent Document 3] Pamphlet of International Publication WO 2003/057670
[Patent Document 4] Specification of US Patent Application Publication No. 2007/0105862
[Patent Document 5] Pamphlet of International Publication WO 2001/007020
[Patent Document 6] Pamphlet of International Publication WO 2006/041874
[Patent Document 7] Specification of EP Patent Application Publication No. 563001
[Patent Document 8] Specification of EP Patent Application Publication No, 616807
[Patent Document 9] Specification of US Patent No. 5554630
[patient Document 10] Pamphlet of International Publication WO 96/033194
[Patent Document 11] Pamphlet of International Publication WO 98/017651
[Patent Document 12] Pamphlet of International Publication WO 98/034923

[Patent Document 13] Japanese Unexamined Patent Application Publication No. 7-133224
[Patent Document 14] Pamphlet of International Publication WO 97/033872
[Patent Document 15] Japanese Unexamined Patent Application Publication No. 2002-193947
[Patent Document 16] Pamphlet of International Publication WO 2008/006790

Non-Patent Document

[Non-Patent Document 1] "Cancer Research" 2004, Vol.64, pp 1802-1810
[Non-Patent Document 2] "Molecular and Cellular Endocrinology" 2006, Vol.248, pp 233-235
[Non-Patent Document 3] "The Journal of Medicinal Chemistry" 1998, Vol.41, No.27, pp 5457-5465
[Non-Patent Document 4] "Bioorganic and Medicinal Chemistry" 2004, Vol.12, No.15, pp 4009-4015
[Non-Patent Document 5] "Bioorganic and Medicinal Chemistry" 2005, Vol.13, No.24, pp 6598-6608

Summary of the Invention

Problem that the Invention is to Solve

An object of the present invention is to provide a screening method for a compound which is useful as a pharmaceutical having a selective inhibitory activity against 17βHSD type 5 and an agent for treating 17βHSD type 5-related diseases such as prostate cancer.

Means for Solving the Problem

In order to achieve the above object, the present inventors conducted intensive studies, and as a result, established a screening method for a compound which is useful for treating 17βHSD type 5-related diseases, by manifesting a tumor by transplanting tumor cells to an animal to be tested and measuring the level of a hormone produced in the tumor, and thereby completed the present invention.
That is, the present invention relates to a screening method for a compound which is useful for treating 17βHSD type 5-related diseases, which comprises: 1) a process of manifesting a tumor by transplanting tumor cells to an immunodeficient mouse; 2) a process of administering a test substance to the immunodeficient mouse; 3) a process of topically administering a steroid which is a biosynthetic material for a hormone into the tumor; 4) a process of measuring the hormone concentration in the tumor of the immunodeficient mouse and calculating the inhibition rate in conversion of the steroid which is a biosynthetic material for a hormone to the hormone; and 5) a process of selecting a substance which suppresses the hormone production in the tumor.
Further, the present invention relates to the screening method for a compound which is useful for treating 17βHSD type 5-related diseases, in which the steroid is dissolved into a gel-type substance for the topical administration in the above step 3).
In addition, the present invention relates to a compound which is useful for treating 17βHSD type 5-related diseases and which is selected by the above screening method or a salt thereof. Further, the present invention relates to a pharmaceutical which comprises a compound which is useful for treating 17βHSD type 5-related diseases and which is selected by the above screening method or a salt thereof, as an active ingredient; a method for treating 17βHSD type 5-related diseases which uses the above compound or a salt thereof; and use of the above compound or a. salt thereof for the manufacture of an agent for treating 17βHSD type 5-related diseases.

Effects of the Invention

According to the screening method of the present invention, it is possible to efficiently evaluate candidate substances which can be agents for treating 17βHSD type 5-related diseases and/or agents for treating 17βHSD type 5-related cancer such as prostate cancer. Also, it is possible to obtain test results with excellent reproducibility and small irregularity by dissolving a steroid which is a biosynthetic material for a hormone such as testosterone in a gel-type substance, and topically administering it into the tumor.

Mode for Carrying out the Invention

Hereinafter, the present invention will be described in detail.
According to the present invention, an embodiment regarding the screening method which comprises manifesting a

tumor by transplanting tumor cells to an animal to be tested and measuring the level of a hormone produced in the tumor is as follows.
A screening method which includes

1) a process of manifesting a tumor by transplanting tumor cells, for example, tumor cells derived from prostate cancer or breast cancer, preferably tumor cells derived from prostate cancer, to an immunodeficient mouse;
2) a process of administering a test substance to the immunodeficient mouse;
3) a process of topically administering a steroid which is a biosynthetic material for a hormone, for example, testosterone or estrogen, preferably testosterone, into the tumor;
4) a process of measuring the hormone concentration, for example, the estrogen concentration or testosterone concentration, in the tumor of the immunodeficient mouse and calculating the inhibition rate in conversion of the steroid which is a biosynthetic material of a hormone to the hormone; and
5) a process of selecting a substance which suppresses the hormone production in the tumor, for example, the production of testosterone or estrogen, preferably the production of testosterone.

1) Process of manifesting a tumor by transplanting tumor cells to an immunodeficient mouse

Examples of the immunodeficient mouse include nude mouse or the like. Sex, age in weeks, weight, and the like of the immunodeficient mouse are not particularly limited as long as the mouse is applicable to the subject screening. Examples of the tumor cells include tumor cells derived from prostate cancer or breast cancer, preferably tumor cells derived from prostate cancer.
Examples of the tumor cells derived from prostate cancer include CWR22, CWR22R ("Cancer Research" 1996, Vol.56, pp 3042-3046), or substrains thereof. Examples of the tumor cells derived from breast cancer include MCF-7 ("Journal of Steroid Biochemistry, 1988, Vol.30, pp 311-314). These tumor cells can be obtained by being excised from, for example, a tumor-bearing immunodeficient mouse.
The tumor cell solution used for the transplant can be obtained by stirring the obtained tumor in a general medium for cultured cells to which a protease is added and performing centrifugation. When the cell solution is diluted by using a gel-type substance such as collagen, Matrigel (trademark), silicon glue, or gelatin, preferably using Matrigel, it has a preferable influence on cell growth after the transplant. It is preferable to adjust the cell concentration after dilution to $1\times10^6$ to $1\times10^8$ cells/mL.
Transplant of the tumor cell solution is performed by subcutaneously injecting the tumor cell solution prepared in the above-described manner to the mouse's back where the tumor cell is located. Preferably, the number of the cells to be transplanted is $1\times10^5$ to $1 \times 10^7$ cells per site, and this can be accomplished by injecting about 0.1 my of the tumor cell solution per site.
"Manifesting'" refers to a state where the mouse after transplant above is raised for a certain period of time, and its tumor site is enlarged to a tumor volume of $10mm^3$ or more.

2.) Process of administering a test substance to the immunodeficient mouse

The test substance used for the screening of the present invention is not particularly limited, and examples thereof include known or novel synthetic compounds, peptides, proteins, natural ingredients derived from plants or marine life, tissue extracts from warm-blooded mammals, and cell culture supernatant. Preferable examples thereof include the compounds having an inhibitory activity against 17βHSD type 5, which are disclosed in the present specification.
The administration of the test substances used in the screening method of the present invention is performed by, for example, an oral administration, an intravenous administration, and a transdermal administration, according to properties of the test substances. When the test substances are administered orally, a method of dissolving the substances in water or organic solvents so as to liquefy them, and then performing a forced administration to an animal with a syringe, a pipet or the like is preferable. For the purpose of comparison and contrast with the screening method of the present invention, a control group to which only the solvents for administering the test substances are administered is used.

3) Process of topically administering a steroid which is a biosynthetic material for a hormone into the tumor

Examples of the hormones include testosterone or oestrogen, and preferably include testosterone.
The steroid which is a biosynthetic material for testosterone is not particularly limited, and examples thereof include androstenedione, dehydroepiandrosterone and the like. The steroid which is a biosynthetic material for estrogen is not particularly limited, and examples thereof include androstenedione, testosterone, estrone, and estrone sulfate.
The above mentioned steroid is dissolved or suspended, preferably dissolved into one or two or more kinds of an

appropriate amount of organic solvents, surfactants, water-soluble solvents, and gel-type substances so as to become a liquid for administration. The organic solvents are not particularly limited, and examples thereof include N,N-dimethylformamide (DMF), dimethylsulfoxide (DMSO) and the like. The surfactants are not particularly limited, and examples thereof include polysorbate 80, Tween and the like. The water-soluble solvents are not particularly limited, and examples thereof include physiological saline and phosphate buffered saline (PBS) and the like. The gel-type substances are not particularly limited, and examples thereof include collagen, Matrigel (trademark), silicon glue, gelatin and the like, preferably collagen or Matrigel, and more preferably Matrigel. By using gel-type substances such as Matrigel, diffusion of administered steroid out of the tumor is thought to be suppressible, and thereby it is possible to obtain test results with excellent reproducibility and small irregularity.
The amount of the steroid for topical administration is 0.1 to 1000 ng, and preferably 0.3 to 30 ng, based on 100 $mm^3$ of tumor volume.
The volume of the liquid for administration is preferably 1/50 to 1/3 of tumor volume.
"Topical administration" refers to the administration of the liquid for administration above to the tumor of the immunodeficient mouse to which the test substance has been administered in the process 2), by a method such as injection. Generally, administration is performed 0.1 to 24 hours before the tumor excision performed in the next process 4).

4) Process of measuring the hormone concentration in the tumor of the immunodeficient mouse and calculating the inhibition rate in conversion of the steroid which is a biosynthetic material for a hormone to the hormone

As described above, examples of the hormone include testosterone or estrogen, preferably testosterone.
The hormone concentration in the tumor can be measured by the following method. A sample for measuring the hormone concentration in the tumor can be obtained by excision of the tumor from the immunodeficient mouse and extraction of hormone-containing ingredients through a general method.
The hormone concentration in the thus obtained sample can be measured by general methods such as an ELISA method.
A sample for measuring background values of the hormone concentration can be obtained by excising the tumor from the mouse to which only the solvent for administering a test substance has been administered, adding the liquid for administering a steroid which is a biosynthetic material of a hormone under ice cooling, then extracting hormone-containing ingredients through the same method as described above.
The inhibition rate is calculated by the following formula.

Inhibition rate (%)=100×(average value of the hormone concentration in the solvent for administering test substance-administered group - average value of the hormone concentration in the test substance-administered group)/(average value of the hormone concentration in the solvent for administering test substance-administered group - average value of the background hormone concentration)

5) Process of selecting a substance which suppresses the hormone production in the tumor

As described above, examples of the hormones include testosterone, or estrogen, preferably testosterone.
Based on the inhibition rate calculated through the above calculation formula as an index, it is possible to select a substance which suppresses the hormone production in the tumor. Substances to be selected is a substance having preferably 30% or more of the maximum inhibition rate upon administration of 1 mg/kg to 300 mg/kg, and more preferably 50% or more of the maximum inhibition rate.
In the screening method of the present invention, the compound of formula (1) can be used.

[Chem. 1]

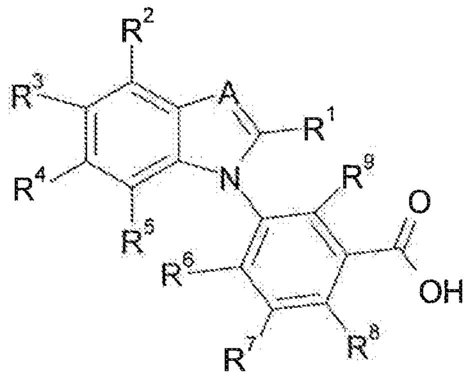

(I)

wherein in formula (I):

A represents C-$R^{10}$ or N;
$R^1$ represents hydrogen, lower alkyl, halogeno lower alkyl, lower alkyl substituted with lower alkyl-O-, lower alkyl-O-, halogeno lower alkyl-O-, lower alkyl-O- substituted with lower alkyl-O-, cycloalkul-L- which may be substituted, aryl-L- which may be substituted, or a heterocyclic group-L- which may be substituted;
L represents a bond, lower alkylene, lower alkenylene, O, lower alkylene-O, or O-lower alkylene;
$R^2$, $R^4$, $R^5$, $R^6$, $R^7$, $R^8$, and $R^9$, which are the same or different from each other, represent hydrogen, lower alkyl, halogen, cyano, lower alkenyl, halogeno lower alkyl, lower alkyl-O-, cyano lower alkyl-O-, halogeno lower alkyl-O-, cycloalkyl, aryl, heteroaryl, aryl-O-, heteroaryl-O-, aryl-lower alkylene-, heteroaryl-lower alkylene-, acyl, acyl-O-, heteroaryl-lower alkylene-O-, lower alkylsulfanyl, amino, hydroxy, sulfanyl, mono-lower alkylamino, di-lower alkylamino, acylamino, or arylamino;
$R^3$ represents hydrogen, halogen, cyano, nitro, halogeno lower alkyl, or lower alkyl-O-;
$R^{10}$ represents hydrogen, aryl which may be substituted, or lower alkyl which may be substituted with hydroxy or lower alkyl-O-,
provided that,
when A is C-$R^{10}$), $R^1$ represents hydrogen or lower alkyl;
when A is N, and $R^1$, $R^2$, $R^4$, $R^5$, $R^6$, $R^7$, $R^8$, and $R^9$ are hydrogen, $R^3$ represents halogen, cyano, halogeno lower alkyl, or lower alkyl-O-;
when A is N, $R^1$ is methyl, and $R^2$ $R^3$ $R^4$, $R^5$, $R^6$, $R^7$, and $R^9$ are hydrogen, $R^8$ is a group other than [(2E)-3-(2-naphthalenyl)-1-oxo-2-buten-1-yl]amino,
The meanings of the symbols in the chemical formula are the same hereinafter.

The compound of formula (1) inhibits 17βHSD type 5 selectively. Accordingly, the compound of formula (1) is useful as an agent for preventing and/or treating "17βHSD type 5-related diseases", for example, diseases associated with androgen and/or estrogen, since androgen and/or estrogen synthesis is suppressed by the inhibition of 17βHSD type 5. Examples of the androgen- and/or estrogen-related diseases include prostate cancer, benign prostatic hyperplasia, acne, seborrhea, hirsutism, baldness, alopecia, precocious puberty, adrenal hypertrophy, polycystic ovary syndrome, breast cancer, endometriosis, leiomyoma and the like. In addition, since AKR1C3 (another name for 17βHSD type 5), which is a subtype of aldo-keto reductase (AKR), metabolizes Polycyclic Aromatic Hydrocarbon (PAH) to generate reactive oxygen species (ROS) and since single nucleotide polymorphism (SNP) of AKR1C3 gene relating to oxidation stress correlates to the risk of lung cancer, lung cancer is also exemplified as 17βHSD type 5-related diseases. The compound of formula (1) is therefore useful as an agent for treating and/or an agent for preventing these diseases.
In addition, since 17βHSD type 5 is considered to be responsible for intracrine androgen synthesis in the prostate, selective inhibitors of 17βHSD type 5 are expected to suppress intracrine androgen synthesis in the prostate selectively. Accordingly, the compound of formula (1) is particularly useful as an agent for preventing and/or treating diseases associated with androgen in the prostate, that is, prostate cancer and benign prostatic hyperplasia.
Further, an embodiment of the compound of formula (1) is the compound of formula (II).

[Chem. 2]

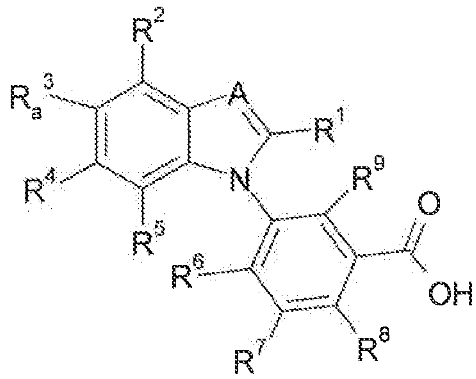

(II)

wherein in formula (II):

A represents C-$R^{10}$ or N;
$R^1$ represents hydrogen, lower alkyl, halogeno lower alkyl, lower alkyl substituted with lower alkyl-O-, lower alkyl-O-, halogeno lower alkyl-O-, lower alkyl-O- substituted with lower alkyl-O-, cycloaikyl-L- which may be substituted, aryl-L- which may be substituted, or a heterocyclic group-L- which may be substituted;
L represents a bond, lower alkylene, lower alkenylene, O, lower alkylene-O, or O-lower alkylene;
R2, $R^4$, $R^5$, $R^6$, $R^7$, $R^8$, and $R^9$, which are the same or different from each other, represent hydrogen, lower alkyl, halogen, cyano, lower alkenyl, halogeno lower alkyl, lower alkyl-O-, cyano lower alkyl-O-, halogeno lower alkyl-O-, cycloalkyl, aryl, heteroaryl, aryl-O-, heteroaryl-O-, aryl-lower alkylene-, heteroaryl-lower alkylene-, acyl, acyl-O-, heteroaryl-lower alkylene-O-, lower alkylsulfanyl, amino, hydroxy, sulfanyl, mono-lower alkylamino, di-lower alkylamino, acylamino, or arylamino;
$R_a^3$ represents hydrogen, halogen, cyano, halogeno lower alkyl, or lower alkyl-O-;
$R^{10}$ represents hydrogen, aryl which may be substituted, or lower alkyl which may be substituted with hydroxy or lower alkyl-O-,
provided that,
when A is C-$R^{10}$, $R^1$ represents hydrogen or lower alkyl, but when $R^1$ $R^2$ $R^5$, $R^6$, $R^7$, $R^8$, and $R^9$ are hydrogen, $R^4$ is chloro, and $R^{10}$ is isobutyl, $R_a^3$ is a group other than hydrogen;
when A is N, and $R^1$, $R^2$, $R^4$, $R^5$, $R^6$, $R^7$, $R^8$, and $R^9$ are hydrogen, $R_a^3$ represents halogen, cyano, or lower alkyl-O-;
when A is N, $R^1$ is methyl, $R^2$, $R^4$, $R^5$ $R^6$, $R^7$, and $R^9$ are hydrogen, and $R^8$ is [(2E)-3-(2-naphthalenyl)-1-oxo-2-buten-1-yl]anmino, $R_a^3$ is a group other than hydrogen;
when A is N, and $R^1$, $R^2$, $R^5$, $R^6$, $R^7$, $R^8$, and $R^9$ are hydrogen, and $R^4$ is tert-butyl, $R_a^3$ is a group other than hydrogen;
when A is N, $R^1$ is hydrogen or methyl, $R^2$, $R^6$, and $R^7$ are hydrogen, $R^4$ and $R^8$ are hydroxy, and $R^5$ is carboxy, $R_a^3$ is a group other than hydrogen.
The meanings of the symbols are the same hereinafter.

Details about the compound of formula (I) and/or formula (II) are explained below. The compound of formula (II) is encompassed by the compound of formula (I), and is one of preferred embodiments of the compound of formula (I), the compound of formula (I) is mainly explained below.

Various preferred examples of the definitions included in the scope of the present invention, in the description above and below in the present specification will be described in detail below.
The term "lower" means a group containing 1 to 10 carbon atoms (hereinafter, referred to also as "$C_{1-10}$"), unless otherwise particularly noted.

The "lower alkyl" means linear or branched alkyl containing 1 to 10 carbon atoms, preferably $C_{1-6}$ alkyl, and for example, methyl, ethyl, propyl, isopropyl, butyl, isobutyl, tert-butyl, pentyl, hexyl group and the like.
The "lower alkylene," means a divalent group formed by removing a hydrogen atom from the "lower alkyl".

The "lower alkenyl" means an unsaturated linear or branched noncyclic hydrocarbon containing 2 to 10 carbon atoms having at least one double bond. The number of carbon atoms is preferably 2 to 6. For example, vinyl, propenyl, butenyl, pentenyl, hexenyl and the like are included.
The "lower alkenylene" means a divalent group formed by removing a hydrogen atom from the "lower alkenyl".

The "halogen" includes F, Cl, Br, or I and is preferably F, Cl, or Br.

The "halogeno lower alkyl" is lower alkyl substituted with one or more halogens. In another embodiment, it means lower alkyl substituted with 1 to 10 halogens. For example, fluoromethyl, chloromethyl, difluoromethyl, dichloromethyl, dibromomethyl, trifluoromethyl, trichloromethyl, fluoroethyl, chloroethyl, 2,2,2-trifluoroethyl, 2,2,2-trichloroethyl, 2,2,3,3,3-pentafluoropropyl, fluoropropyl, fluorobutyl, fluorohexyl and the like are included.

The "cycloalkyl" is a saturated hydrocarbon ring group containing 3 to 10 carbon atoms and optionally having a bridge. For example, cyclopropyl, cyclobutyl, cyclopentyl, cyclohexyl, cycloheptyl, norbornyl, adamantyl group and the like are included.

The "aryl" is a $C_{6-14}$ monocyclic to tricyclic aromatic hydrocarbon ring group which contains a ring group condensed with $C_{5-8}$ cycloalkene at the double bond site thereof. For example, phenyl, naphthyl, tetrahydronaphthalenyl, indenyl, fluorenyl group, and the like are included. In another embodiment, it is phenyl, naphthyl, or anthryl. In yet another embodiment, it is phenyl.

The "heterocyclic" group means 3 to 6-membered monocyclic heteroaromatic ring groups having preferably 1 to 4 nitrogen atoms, for example, pyrrolyl, imidazolyl, pyrazolyl, pyridyl, pyrimidinyl, pyrazinyl, pyridazinyl, triazolyl (for example, 4H-1,2,4-triazolyl, 1H-1,2,3-triazolyl, 2H-1,2,3-triazolyl and the like), tetrazolyl (for example, 1H-tetrazolyl, 2H-tetrazolyl and the like) and the like; condensed heteroaromatic ring groups having 1 to 5 nitrogen atoms, for example, indolyl, isoindolyl, indolizinyl, benzimidazolyl, quinolyl, isoquinolyl, indazolyl, benzotriazolyl, tetrazolopyridazinyl (for example, tetrazolo[1,5-b]pyridazinyl and the like), quinoxalinyl, imidazopyridyl (for example, imidazo[1,2-a]pyridyl and the like) and others; 3 to 6-membered monocyclic heteroaromatic ring groups having one oxygen atom, for example, pyranyl, furyl and the like; 3 to 6-membered monocyclic heteroaromatic ring groups having 1 to 2 sulfur atoms, for example, thienyl and the like; 3 to 6-membered monocyclic heteroaromatic ring groups having 1 to 2 oxygen atoms and 1 to 3 nitrogen atoms, for example, oxazolyl, isooxazolyl, oxadiazolyl (for example, 1,2,4-oxadiazolyl, 1,3,4-oxadiazolyl, 1,2,5-oxadiazolyl and the like) and others; condensed heteroaromatic ring groups having 1 to 2 oxygen atoms and 1 to 3 nitrogen atoms, for example, benzofurazanyl, benzoxazolyl, benzoxadiazolyl and the like; 3 to 6-membered monocyclic heteroaromatic ring groups having 1 to 2 sulfur atoms and 1 to 3 nitrogen atoms, for example, thiazolyl, thiadiazolyl (for example, 1,2,4-thiadiazolyl, 1,3,4-thiadiazolyl, 1,2,5-thiadiazolyl and the like) and others; condensed heteroaromatic ring groups having 1 to 2 sulfur atoms and 1 to 3 nitrogen atoms, for example, benzothiazolyl, benzothiadiazolyl, imidazothiazolyl (for example, imidazo[2,1-b][1,3]thiazolyl and the like) and others; condensed heteroaromatic ring groups having 1 to 2 oxygen atoms, for example, benzofuranyl, dibenzo[b,d]furanyl and the like; condensed heteroaromatic ring groups having 1 to 2 sulfur atoms, for example, benzothienyl and the like; and others.

The "heterocyclic" group further means a 3 to 10-membered saturated or unsaturated ring group containing 1 to 4 hetero atoms selected from O, N and S, and preferable examples thereof include 3 to 7-membered saturated heteromonocyclic groups having 1 to 4 nitrogen atoms, for example, pyrrolidinyl, imidazolidinyl, piperidyl, piperazinyl, and the like; 3 to 10-membered saturated or unsaturated bicyclic heterocyclic groups having 1 to 4 nitrogen atoms, for example, quinuclidinyl and the like; 3 to 6-membered saturated heteromonocyclic groups having one oxygen atom, for example, 1H-tetrahydropyranyl, tetrahydrofuranyl and the like; 3 to 6-membered saturated or unsaturated heteromonocyclic groups having 1 to 2 oxygen atoms and 1 to 3 nitrogen atoms, for example, morpholinyl, oxazolinyl (for example, 2-oxazolinyl and the like) and others; 3 to 6-membered saturated or unsaturated heteromonocyclic groups having 1 to 2 sulfur atoms and 1 to 3 nitrogen atoms, for example, thiazolidinyl and others; and the like.

The "heteroaryl" means a group included in the "heterocyclic" group and having an aromaticity.

The "cyano lower alkyl" is lower alkyl substituted with one or more cyano groups. In another embodiment, it means lower alkyl substituted with 1 to 3 cyano groups. For example, cyanomethyl, cyanoethyl, 2,3-dicyanopropyl, and the like are included.

The "acyl group" include, for example, carboxy; esterified carboxy; carbamoyl substituted with lower alkyl, aryl, aryl-lower alkylene, arylsulfonyl, sulfonyl substituted with heterocyclic group, lower alkylsulfonyl or heterocyclic group; substituted or unsubstituted arylsulfonyl; sulfonyl substituted with a heterocyclic group; lower alkylsulfonyl; cycloalkylcarbonyl; lower alkyl-CO~; HCO-; substituted or unsubstituted aryl-CO-; carbonyl substituted with a heterocyclic group, and the like.

The esterified carboxy group include substituted or unsubstituted lower alkyl-O-C(=O)- (for example, methoxycarbonyl, ethoxycarbonyl, propoxycarbonyl, butoxycarbonyl, tert-butoxycarbonyl, hexyloxycarbonyl, 2-iodoethoxycarbonyl, 2,2,2-trichloroethoxycarbonyl and the like), substituted or unsubstituted aryl-O-C(=O)- (for example, phenoxycarbonyl, 4-nitropbenoxycarbonyl, 2-naphthyloxycarbonyl and the like), substituted or unsubstituted aryl-lower alkylene-O-C(=O)- (for example, benzyloxycarbonyl, phenethyloxycarbonyl, benzhydryloxycarbonyl, 4-nitrobenzyloxycarbonyl and the like) and others.

The carbamoyl group substituted with lower alkyl include methylcarbamoyl, ethylcarbamoyl, propylcarbamoyl, dimethylcarbamoyl, diethylcarbamoyl, methylethylcarbamoyl and the like.

The carbamoyl group substituted with aryl include phenylcarbamoyl, naphthylcarbamoyl, phenylcarbamoyl substituted with lower alkyl (for example, tolylcarbamoyl, xylylcarbamoyl and the like) and others.

The carbamoyl group substituted with aryl-lower alkylene include benzylcarbamoyl, phenethylcarbamoyl, phenylpropylcarbamoyl and the like.

The carbamoyl group substituted with arylsulfonyl include phenylsulfonylcarbamoyl, tolylsulfonylcarbamoyl and the like.

The carbamoyl substituted with (sulfonyl substituted with a heterocyclic group) include pyridylsulfonylcarbamoyl and the like.

The carbamoyl group substituted with lower alkylsulfonyl include methylsulfonylcarbamoyl, ethylsulfonylcarbamoyl and the like.

The substituted or unsubstituted arylsulfonyl group include phenylsulfonyl, tolylsulfonyl, halophenylsulfonyl (for example, fluorophenylsulfonyl and the like) and others.

The sulfonyl group substituted with a heterocycle include pyrrolidinylsulfonyl and the like.

The lower alkylsulfonyl group include methylsulfonyl, ethylsulfonyl and the like.

The cycloalkylcarbonyl group include, for example, cyclopropylcarbonyl, cyclobutylcarbonyl, cyclopentylcarbonyl or cyclohexylcarbonyl and the like.

The lower alkyl-CO- group include acetyl, propionyl, butyryl, isobutyryl, valeryl, isovaleryl, pivaloyl, hexanoyl and the like.

The substituted or unsubstituted aryl-CO- group include benzoyl, naphtoyl, toluoyl, di(tert-butyl)benzoyl, halogeno lower alkyl-O-benzoyl (for example, trifluoromethoxybenzoyl and the like) and others.

The heterocyclic group moiety of the "carbonyl substituted with a heterocyclic group" means the "heterocyclic" group described above. For example, pyridylcarbonyl and the like are included.

The "mono-lower alkylamino" means an amino group substituted with a "lower alkyl" group described above. For example, methylamino, ethylamino, propylamino, isopropylamino, butylamino, isobutylamino, pentylamino, hexylamino, and the like are included.

The "di-lower alkylamino" means an amino group substituted with two same or different "lower alkyl" described above. For example, dimethylamino, diethylamino, dipropylamino, diisopropylamino, dibutylamino, diisobutylamino, dipentylamino, dihexylamino, ethylmethylammo, methylpropylamino, butylmethylamino, ethylpropylamino, butylethylamino and the like are included.

The "which may be substituted" means unsubstituted or substituted with 1 to 5 substituents, The "substituted" means that having 1 to 5 substituents. Further, if there are multiple substituents, the substituents may be the same or different from each other.

The substituents for the "cycloalkyl which may be substituted", "aryl which may be substituted" or "heterocyclic group which may be substituted" in of $R^1$ and $R^{10}$ include, for example, lower alkyl, halogen, cyano, lower alkenyl, cycloalkyl, halogeno lower alkyl, lower alkyl-O-, cyano lower alkyl-O-, halogeno lower alkyl-O-, aryl, heteroaryl, aryl-O-, heteroaryl-O-, aryl-lower alkylene-, acyl, acyl-O-, heteroaryl-lower alkylene-O-, lower alkylsulfanyl, nitro, amino, hydroxy, sulfanyl, mono-lower alkyl amino, di-lower alkylamino, acylamino, and arylamino group. In another embodiment, a group selected from lower alkyl, halogen, cyano, lower alkenyl, halogeno lower alkyl, lower alkyl-O-, cyano lower alkyl-O-, halogeno lower alkyl-O-, and lower alkyl-CO-, is exemplified. In yet another embodiment, a group selected from lower alkyl, halogen, halogeno lower alkyl, lower alkyl-O-, and lower alkyl-CO- is exemplified.

Although "cycloalkyl", "phenyl", "cyclohexyl" and the like are described as monovalent groups in the present specification for convenience, they may be multivalent groups of divalent or higher valency according to their structures. The compound which can be used in the screening method of the present invention encompasses these structures. Specific embodiments of the divalent group correspond to those having the suffix of the above ring groups converted into diyl in accordance with the Nomenclature of Organic Chemistry. For example, a divalent group corresponding to a phenyl group that is a monovalent group is a phenylene. A divalent group corresponding to a benzothiazolyl group that is a monovalent group is benzothiazolediyl.

The "selective to 17βHSD type 5" and "selective to AKR1C3" means that the inhibitory activity against 17βHSD type 3 and AKR1C2 shows the value of 3-fold or more, preferably 10-fold or more, and more preferably 100-fold or more, in terms of $IC_{50}$ value, compared to the inhibitory activity against human 17βHSD type5(AKR1C3).

An embodiment of the compound of formula (I), which can be used in the screening method of the present invention, is shown below.

(1) A compound of formula (II).
(2) The compound wherein A is C-$R^{10}$.
(3) The compound wherein A is N.
(4) The compound wherein $R^1$ is hydrogen.
(5) The compound wherein $R^1$ is lower alkyl.
(6) The compound wherein $R^1$ is halogeno lower alkyl.
(7) The compound wherein $R^1$ is lower alkyl substituted with lower alkyl-O-.
(8) The compound wherein $R^1$ is lower alkyl substituted with phenyl wherein the phenyl may be substituted with a

group selected from lower alkyl, halogen, halogeno lower alkyl, and lower alkyl-O-.
(9) The compound wherein $R^1$ is lower alkyl substituted with phenyl-O-, wherein the phenyl may be substituted with a group selected from lower alkyl, halogen, halogeno lower alkyl, and lower alkyl-O-.
(10) The compound wherein $R^1$ is lower alkyl-O- which may be substituted with phenyl.
(11) The compound wherein $R^1$ is cycloalkyl.
(12) The compound wherein $R^1$ is phenyl which may be substituted with a group selected from lower alkyl, halogen, and lower alkyl-O-.
(13) The compound wherein $R^2$ is hydrogen.
(14) The compound wherein $R^4$ is hydrogen.
(15) The compound wherein $R^5$ is hydrogen.
(16) The compound wherein $R^6$ is hydrogen.
(17) The compound wherein $R^7$ is hydrogen.
(18) The compound wherein $R^8$ is hydrogen.
(19) The compound wherein $R^9$ is hydrogen.
(20) The compound wherein $R^2$, $R^4$, $R^5$, $R^6$, $R^7$, $R^8$, and $R^9$, which are the same or different from each other, are hydrogen or halogen.
(21) The compound wherein $R^3$ or $R_a{}^3$ is halogeno lower alkyl or cyano.
(22) The compound wherein $R^3$ or $R_a{}^3$ is halogeno lower alkyl.
(23) The compound wherein $R^3$ or $R_a{}^3$ is cyano.
(24) The compound wherein $R^3$ or $R_a{}^3$ is trifluoromethyl.
(25) The compound which is a combination of any two or more of (1) to (24) above.

Specific examples of the compound of the above (25) include the following compounds.

(26) A compound of formula (II) wherein A is C-$R^{10}$.
(27) The compound described in (26), wherein $R^1$ is hydrogen, and $R^2$, $R^4$, $R^5$, $R^6$, $R^7$, $R^8$, and $R^9$, which are the same or different from each other, are hydrogen or halogen.
(28) The compound described in (27), wherein $R_a{}^3$ is halogeno lower alkyl, or cyano.
(29) A compound of formula (II) wherein A is N.
(30) The compound described in (29), wherein $R^2$. $R^4$, $R^5$, $R^6$, $R^7$, $R^8$, and $R^9$, which are the same or different from each other, are hydrogen or halogen.
(31) The compound described in (30), wherein $R_a{}^3$ is halogeno lower alkyl, or cyano.
(32) The compound described in (31) above, wherein $R_a{}^3$ is trifluoromethyl.
(33) The compound described in any of (29) to (32), wherein $R^1$ represents hydrogen; lower alkyl; halogeno lower alkyl; lower alkyl substituted with lower alkyl-O-, phenyl or phenyl-O-; phenyl which may be substituted with lower alkyl, halogen, or lower alkyl-O-; or cycloalkyl, wherein the phenyl moiety in the lower alkyl substituted with phenyl or phenyl-O- may be substituted with lower alkyl, halogen, halogeno lower alkyl, or lower alkyl-O-.
(34) The compound described in (32), wherein $R^1$ is lower alkyl substituted with hydrogen, lower alkyl, halogeno lower alkyl, or lower alkyl-O-.
(35) The compound described in (32), wherein $R^1$ is lower alkyl-O- which may be substituted with phenyl.
(36) The compound described in (32), wherein $R^1$ is cycloalkyl.
(37) The compound described in (32), wherein $R^1$ is phenyl which may be substituted with a group selected from lower alkyl, halogen, and lower alkyl-O-.
(38) The compound described in (32), wherein $R^1$ is lower alkyl which is substituted with phenyl or lower alkyl substituted with phenyl-O-, wherein the said phenyl may be substituted with a group selected from lower alkyl, halogen, halogeno lower alkyl, and lower alkyl-O-.

Specific compounds included in the compound of formula (I) are the following compounds.
3-(5-cyano-1H-indol-1-yl)benzoic acid,
3-[3-phenyl-5-(trifluoromethyl)-1H-indol-1-yl]benzoic acid,
3-[3-(2-methoxyethyl)-5-(trifluoromethyl)-1H-indol-1-yl]benzoic acid,
3-[5-(trifluoromethyl)-1H-benzimidazol-1-yl]benzoic acid,
3-[2-methyl-5-(trifluoromethyl)-1H-benzimidazol-1-yl]benzoic acid,
3-[2-ethyl-5-(trifluoromethyl)-1H-benzimidazol-1-yl]benzoic acid,
3-[2-propyl-5-(trifluoromethyl)-1H-benzimidazol-1-yl]benzoic acid,
3-[2,5-bis(trifluoromethyl)-1H-benzimidazol-1-yl]benzoic acid,
3-[2-phenoxymethyl-5-(trifluoromethyl)-1H-benzimidazol-1-yl]benzoic acid,
3-[2-methoxy-5-(trifluoromethyl)-1H-benzimidazol-1-yl]benzoic acid,

3-[2-cyclopropyl-5-(trifluoromethyl)-1H-benzimidazol-1-yl]benzoic acid,
3-[2-cyclohexyl-5-(trifluoromethyl)-1H-benzimidazol-1-yl]benzoic acid,
3-[2-phenyl-5-(trifluoromethyl)-1H-benzimidazol-1-yl]benzoic acid,
3-[2-(3-methylphenyl)-5-(trifluoromethyl)-1H-benzimidazol-1-yl]benzoic acid,
3-[2-benzyl-5-(trifluoromethyl)-1H-benzimidazol-1-yl]benzoic acid,
3-[2-(3-fluorobenzyl)-5-(trifluoromethyl)-1H-benzimidazol-1-yl]benzoic acid,
3-[2-(4-fluorobenzyl)-5-(trifluoromethyl)-1H-benzimidazol-1-yl]benzoic acid,
3-[2-(4-chlorobenzyl)-5-(trifluoromethyl)-1H-benzimidazol-1-yl]benzoic acid, and
3-[2-(benzyloxy)-5-(trifluoromethyl)-1H-benzimidazol-1-yl]benzoic acid.

The compound of formula (I) may in some cases exist in the form of tautomers or geometrical isomers, depending on the kind of substituents. In the present specification, the compound of formula (I) may be described only in one form of the isomers, but the compound which can be used in the screening method of the present invention includes other isomers as well as isolated forms or mixtures thereof.

Further, the compound of formula (I) may have asymmetric carbon atoms or axial asymmetries in some cases, and correspondingly, it may exist in the form of optical isomers. The compound which can be used in the screening method of the present invention also includes isolates or mixtures of optical isomers of the compound of formula (I).

The compound of formula (I) may be converted into a salt thereof by a general method. The salt of the compound of formula (I) is a pharmaceutically acceptable salt. Examples of the salt include metal salts such as alkali metal salts (for example, sodium salt, potassium salt, and the like) or alkaline earth metal salts (for example, calcium salt, magnesium salt, and the like), ammonium salts, organic base salts (for example, trimethylamine salt, triethylamine salt, pyridine salt, picoline salt, dicyclohexylamine salt, and the like), organic acid salts (for example, acetate, malonate, tartrate, methanesulfonate, benzenesulfonate, formate, toluenesulfonate, trifluoroacetate, and the like), inorganic acid salts (for example, hydrochloride, hydrobromide, sulfate, phosphate, and the like), amino acid salts (for example, salt with arginine, aspartic acid, glutamic acid, and the like) and others. Accordingly, the compound which can be used in the screening method of the present invention encompasses all of the compounds of formula (I) and pharmaceutically acceptable salts thereof.

The compound of formula (I) and a pharmaceutically acceptable salt thereof also include various hydrates or solvates, and polymorphisms. Further, the compound which can be used in the screening method of the present invention includes compounds labeled with various radioactive or non-radioactive isotopes.

Further, the compound of formula (I) also includes a pharmacologically acceptable prodrug thereof. The pharmacologically acceptable prodrug is a compound having a group which can be converted into an amino group, a hydroxyl group, a carboxyl group or the like by solvolysis or under a physiological condition. Examples of the group for forming a prodrug include those as described, for example, in Prog. Med., 5, 2157-2161 (1985) or "pharmaceutical Research and Development" (Hirokawa Publishing Company, 1990), Vol. 7, "Drug Design", 163-198.

(General Production Process)

The compound of formula (I) which can be used in the screening method of the present invention can be produced by utilizing the characteristics based on the types of basic skeleton or substituents and by applying various known synthetic methods. It is sometimes effective, in terms of production techniques, that the functional group is protected by an appropriate protecting group or replaced by a group that can be easily converted into the functional group in the stage of a starting material to intermediate, depending on the type of the functional group during the production. Examples of such functional groups include an amino group, a hydroxy group, a carboxyl group, and the like, and examples of such protecting groups include protecting groups described, for example, in "Protective Groups in Organic Synthesis", (3rd eddition, 1999), edited by T.W. Greene and P.G.M. Wuts. These protecting groups may be appropriately selected and used depending on the reaction conditions. According to such a method, a desired compound can be obtained by introducing the protecting group and carrying out the reaction, and then removing the protecting group, or converting the protecting group into a desired group, if desired.

(Production Process 1)

[Chem. 3]

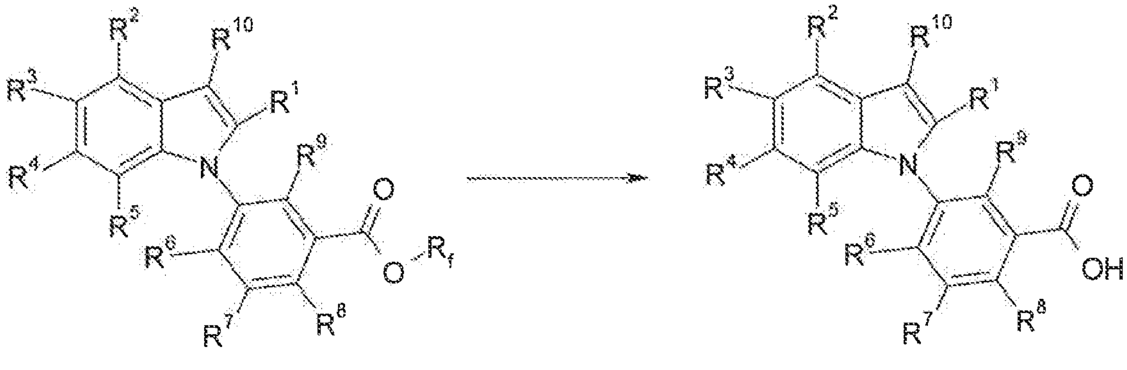

(III) (Ia)

Production Process 1 is a method in which a compound of formula (Ia) is produced by subjecting a compound of formula (III), (wherein in the formula, $R_f$ represents lower alkyl), to hydrolysis.

The hydrolysis may be carried out under either of acidic conditions or basic conditions. The hydrolysis is preferably carried out under basic conditions. The base to be used is preferably an inorganic base such as sodium hydroxide, potassium hydroxide, sodium carbonate, potassium carbonate, cesium carbonate, or the like.

The solvent used is not particularly limited, as long as the solvent does not inhibit the reaction and dissolves the starting materials to a certain level, and examples thereof include alcohols such as methanol, ethanol, and 2-propanol; ethers such as diethyl ether, diisopropyl ether, tetrahydrofuran, dioxane, dimethoxyethane, diethylene glycol dimethyl ether, tert-butyl methyl ether or cyclopentyl methyl ether; or water, or a mixture thereof. Preferred is a mixed solvent of tetrahydrofuran and water.

The reaction temperature of this reaction is generally 0˚C to 100˚C, preferably 0˚C to 50˚C, varying depending on the starting compounds, reagents and the like.

The reaction time of this reaction is generally 5 minutes to 24 hours, preferably 10 minutes to 12 hours, varying depending on the starting compounds, reagents, the reaction temperature and the like.

(Production Process 2)

(Production Process 2-1)

[Chem. 4]

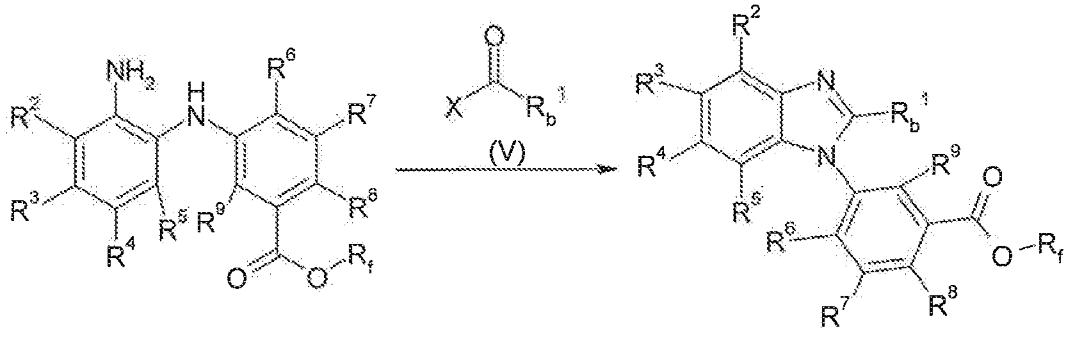

(IV) (VI)

Production Process 2-1 is a method in which a compound of formula (VI) (wherein in the formula, $R_b{}^1$ and $R_f$ are as defined below) is obtained by reacting a compound of formula (IV) (wherein in the formula, $R_f$ represents lower alkyl) and a compound of formula (V) (wherein in the formula, $R_b{}^1$ represents lower alkyl, halogeno lower alkyl, lower alkyl substituted with lower alkyl-O-, cycloalkyl-$L_a$- which may be substituted, aryl-$L_a$- which may be substituted, or a heterocyclic group-$L_a$- which may be substituted; $L_a$ is a bond, lower alkylene, or lower alkenylene; and X represents a

leaving group.)

(Production Process 2-2)

Production Process 2-2 is a method in which a compound of formula (Ib) (wherein in the formula, $R_b{}^1$ is as defined above) is obtained by hydrolysis of the resulting compound of formula (VI).

[Chem. 5]

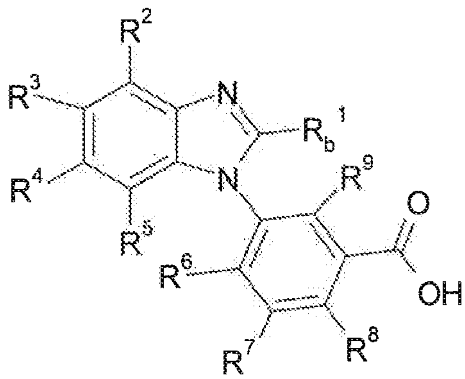

(Ib)

The solvent used in Production Process 2-1 is not particularly limited, as long as the solvent does not inhibit the reaction and dissolves the starting materials to a certain level, and examples thereof include aromatic hydrocarbons such as benzene, toluene or xylene; halogenated hydrocarbons such as dichloromethane, chloroform, carbon tetrachloride, 1,2-dichloroethane, chlorobenzene or dichlorobenzene; ethers such as diethyl ether, diisopropyl ether, tetrahydrofuran, dioxane, dimethoxyethane, diethylene glycol dimethyl ether, tert-butyl methyl ether or cyclopentylmethyl ether; nitro compounds such as nitromethane; nitriles such as acetonitrile or isobutyronitrile; amides such as formamide, N, N-dimethylformamide, N,N-dimethylacetamide or N-methyl-2-pyrrolidinone; sulfoxides such as dimethyl sulfoxide; sulfones such as sulfolane; alcohols such as methanol, ethanol, or 2-propanol; and a mixture thereof. Preferred are ethers, particularly tetrahydrofuran.

Examples of the leaving group X include a halogen atom, a lower alkoxy group, a phenoxy group, an imidazolyl group, and the like.

The reaction temperature of Production Process 2-1 is generally 0˚C to 100˚C, preferably 20˚C to 100˚C, varying depending on the starting compounds, reagents and the like.

The reaction time of Production Process 2-1 is generally 5 minutes to 24 hours, preferably 10 minutes to 12 hours, varying depending on the starting compounds, reagents, the reaction temperature and the like.

Production Process 2-2 may be carried out under the same conditions as hydrolysis of Production Process 1.

Further, the compound of formula (Ib) may be obtained by changing the reaction order of Production Process 2-1 and Production Process 2-2, that is by first hydrolyzing the compound of formula (IV) and then reacting with the compound of formula (V). In this case, respective reaction conditions are as set forth above.

(Production Process 3)

(Production Process 3-1)

[Chem. 6]

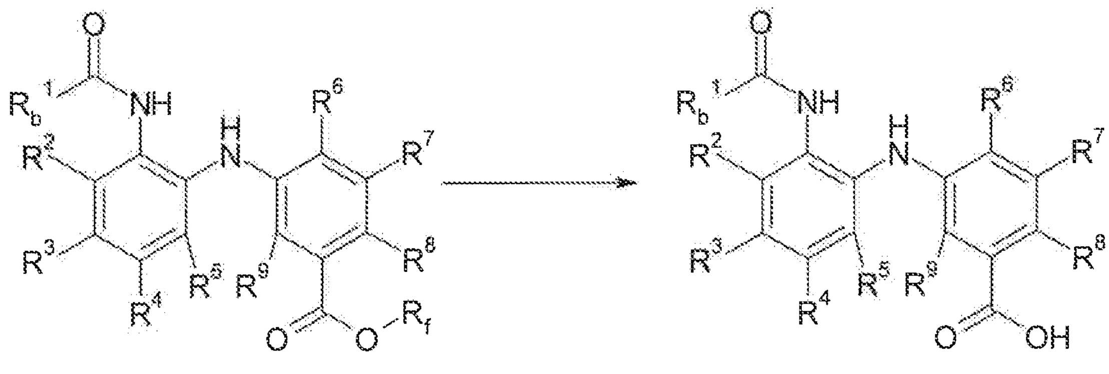

(VII) (VIII)

In Production Process 2-1, the compound of formula (VII) (wherein in the formula, $R_b^1$ and $R_f$ are as defined above) may be isolated, but not the compound of formula (VI). Production Process 3-1 is a method in which a compound of formula (VIII) (wherein in the formula, $R_b1$ is as defined above) is obtained by hydrolyzing a compound of formula (VII).

(Production Process 3-2)

Production Process 3-2 is a method in which a compound of formula (Ib) is obtained by heating the resulting compound of formula (VIII) under acidic conditions.

Production Process 3-1 may be carried out under the same conditions as hydrolysis of Production Process 1.

The solvent used in Production Process 3-2 may suitably be an acidic solvent, for example, acetic acid, trifluoroacetic acid, hydrochloric acid, or the like.

The reaction temperature of Production Process 3-2 is generally 0˚C to 100˚C, preferably 20˚C to 100˚C, varying depending on the starting compounds, reagents and the like.

(Production Process 4)

(Production Process 4-1)

[Chem. 7]

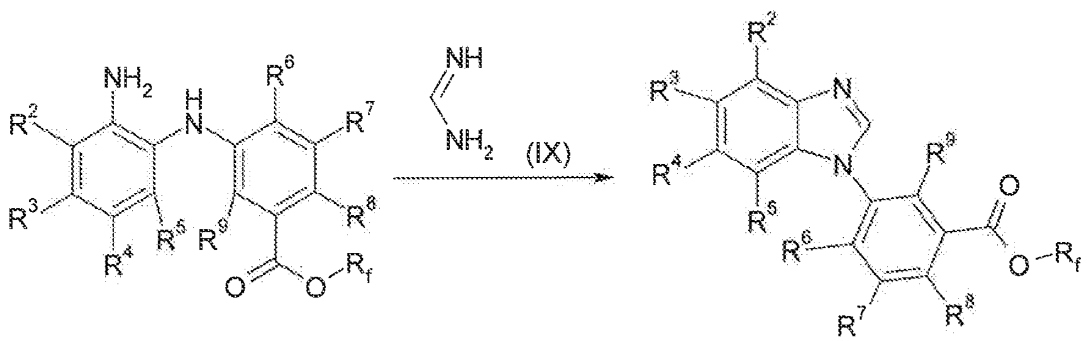

(IV) (X)

Production Process 4-1 is a method in which a compound of formula (X) (wherein in the formula, $R_f$ is as defined above) is obtained by reacting a compound of formula (IV) with a compound of formula (IX) or an acid addition salt of (IX).

(Production Process 4-2)

Production Process 4-2 is a method in which a compound of formula (Ic) is obtained by hydrolizing the resulting compound of formula (X).

[Chem. 8]

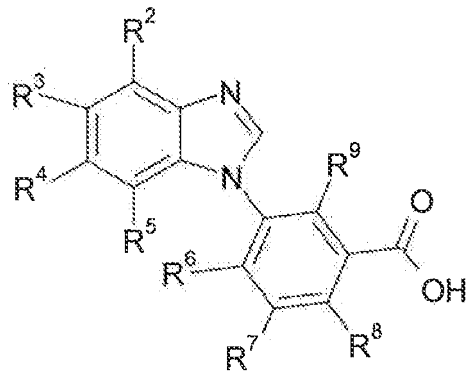

(Ic)

The solvent used in Production Process 4-1 is not particularly limited, as long as the solvent does not inhibit the reaction and dissolves the starting materials to a certain level, and examples thereof include aromatic hydrocarbons such as benzene, toluene or xylene; halogenated hydrocarbons such as dichloromethane, chloroform, carbon tetrachloride, 1,2-dichloroethane, chlorobenzene or dichlorobenzene; ethers such as diethyl ether, diisopropyl ether, tetrahydrofuran, dioxane, dimethoxyethane, diethylene glycol dimethyl ether, tert-butyl methyl ether or cyclopentylmethyl ether; nitro compounds such as nitromethane; nitriles such as acetonitrile or isobutyronitrile; amides such as formamide, N, N-dimethylformamide, N,N-dimethylacetamide or N-methyl-2-pyrroiidinone; sulfoxides such as dimethyl sulfoxide; sulfones such as sulfolane; alcohols such as methanol, ethanol, or 2-propanol; and a mixture thereof. Preferred are alcohols, particularly ethanol.

The reaction temperature of Production Process 4-1 is generally 0˚C to 100˚C, preferably 20˚C to 100˚C, varying depending on the starting compounds, reagents and the like.

The reaction time of Production Process 4-1 is generally 5 minutes to 24 hours, preferably 10 minutes to 12 hours, varying depending on the starting compounds, reagents, the reaction temperature and the like.

Production Process 4-2 may be carried out under the same conditions as hydrolysis of Production Process 1.

(Production Process 5)

(Production Process 5-1)

[Chem. 9]

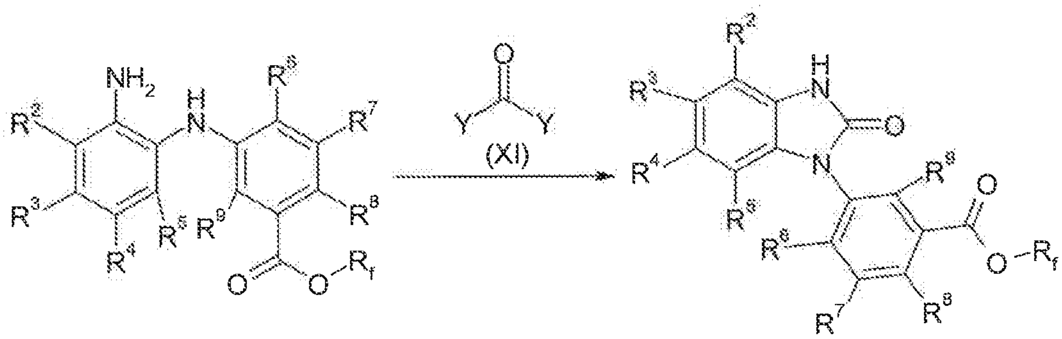


(IV) (XII)

Production Process 5-1 is a method in which a compound of formula (XII) (wherein in the formula, $R_f$ is as defined below) is obtained by reacting a compound of formula (IV) (wherein in the formula, $R_f$ represents lower alkyl) with a compound of formula (XI).

(Production Process 5-2)

[Chem. 10]

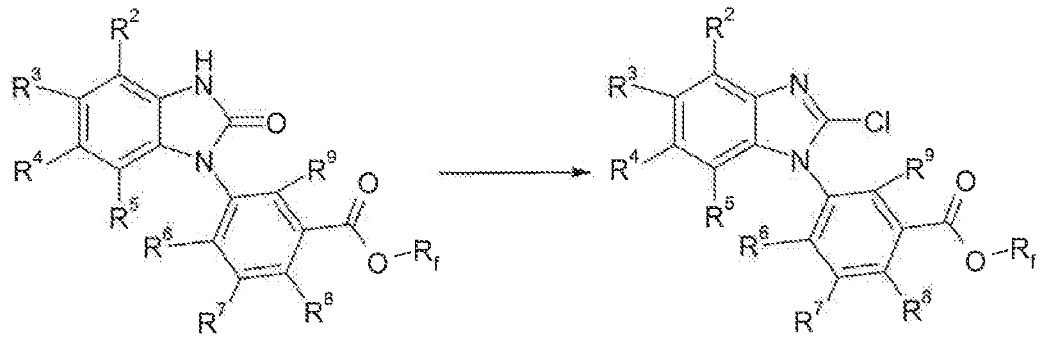

(XII) (XIII)

Production Process 5-2 is a method in which a compound of formula (XIII) (wherein in the formula, $R_f$ is as defined above) is obtained by subjecting the resulting compound of formula (XII) to halogenation.

(Production Process 5-3)

[Chem. 11]

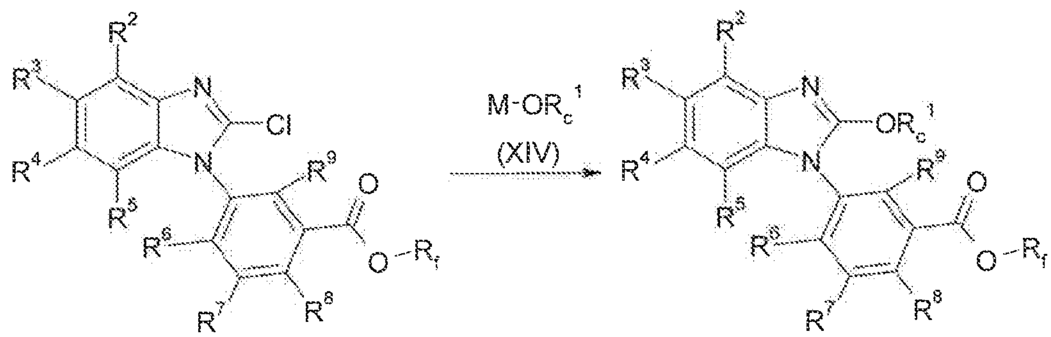


(XIII) (XV)

Production Process 5-3 is a method in which a compound of formula (XV) (wherein in the formula, $R_c^1$ and $R_f$ are as defined below) is obtained by reacting the resulting compound of formula (XIII) with a compound of formula (XIV) (wherein in the formula, $R_c^1$ represents lower alkyl, halogeno lower alkyl, lower alkyl substituted with lower alkyl-O-, cycloalkyl which may be substituted, aryl which may he substituted, or a heterocyclic group which may be substituted; and M represents a metal.)

(Production Process 5-4)

Production Process 5-4 is a method in which a compound of formula (Id) is obtained by hydrolyzing the resulting compound of formula (XV).

[Chem. 12]

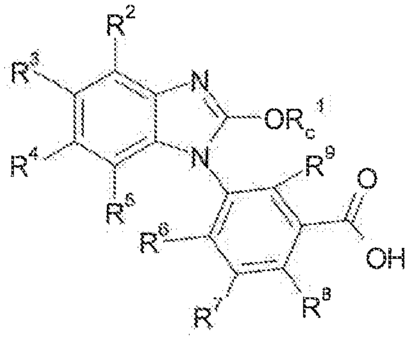

(Id)

Production Process 5-1 may be carried out under the same conditions as in Production Process 2-1. Further, it may be advantageous in some cases for the smooth progress of the reaction to carry out the reaction in the presence of an organic base such as triethylamine, N,N-diisopropyletliylamine, or N-methylmorpholine, or an inorganic base such as potassium carbonate, sodium carbonate, or potassium hydroxide.

Examples of the leaving group Y include a halogen atom, a lower alkoxy group, a phenoxy group, an imidazolyl group, and the like. Preferred is an imidazolyl group.

Production Process 5-2 may be carried out by dissolving the resulting compound of formula (XII) in phosphorus oxychloride or the like, and then adding phosphorus pentachloride or the like if necessary, followed by heating.

The reaction temperature of Production Process 5-2 is generally 0˚C to 120˚C, preferably 20˚C to 100˚C, varying depending on the starting compounds, reagents and the like.

The reaction time of Production Process 5-2 is generally 5 minutes to 24 hours, preferably 10 minutes to 20 hours, varying depending on the starting compounds, reagents, the reaction temperature and the like.

The solvent used in Production Process 5-3 is not particularly limited, as long as the solvent does not inhibit the reaction and dissolves the starting materials to a certain level, and examples thereof include aromatic hydrocarbons such as benzene, toluene or xylene; ethers such as diethylether, diisopropyl ether, tetrahydrofuran, dioxane, dimethoxyethane, diethylene glycol dimethyl ether, tert-butyl methyl ether or cyclopentyl methyl ether; amides such as formamide, N,N-dimethylformamide. N,N-dimethylacetamide or N-methyl-2-pyrrolidinone; sulfoxides such as dimethyl sulfoxide; sulfones such as sulfolane; alcohols such as methanol, ethanol, or 2-propanol; and a mixture thereof. Preferred are alcohols, N,N-dimethylforinamide, or dimethyl sulfoxide.

Examples of the metal M include alkali metals such as lithium, sodium, and potassium, alkaline earth metals such as magnesium, and calcium, or the like.

The reaction temperature of Production Process 5-3 is generally 0˚C to 100˚C, preferably 20˚C to 100˚C, varying depending on the starting compounds, reagents and the like.

The reaction time of Production Process 5-3 is generally 5 minutes to 24 hours, preferably 10 minutes to 20 hours, varying depending on the starting compounds, reagents, the reaction temperature and the like.

Production Process 5-4 may be carried out under the same conditions as hydrolysis of Production Process 1.

Further, the compound of formula (Id) may be obtained by changing the reaction order of Production Process 5-3 and Production Process 5-4, that is, by first hydrolyzing the compound of formula (XIII) and then reacting with the compound of formula (XIV). In this case, respective reaction conditions are as set forth above.

In addition, other compounds of formula (1) may be obtained by applying the methods known to the person skilled in the art, such as ordinary alkylation, esterification, amidation, acylation, and reduction (including reduction of aryl and/or heteroaryl, dehalogenation, and the like), to the compound of formula (1) or a pharmaceutically acceptable salt thereof.

The compound of formula (1) having various functional group(s) can also be produced by applying methods well known to the person skilled in the art or known production processes, or variations thereof. For example, a desired compound can be produced by subjecting the compound obtained by the production process described above to a substituent-modification reaction. Representative reactions are shown below.

(1) Alkylation

Among the compound of formula (I), the compound having a lower alkoxy group or a lower alkylamino group can be produced by subjecting a compound having a hydroxy group or an amino group to an alkylation reaction. For example, the reaction can be carried out by the methods described in "The fourth edition of Courses in Experimental Chemistry (Vol. 20)" edited by The Chemical Society of Japan, Maruzen, 1992 and "The fifth edition of Courses in Experimental Chemistry (Vol. 14)" edited by The Chemical Society of Japan, Maruzen, 2005; or "Compendium of Organic

Synthetic Methods", Vols. 1 to 3, or the like.

(2) Reduction

Among the compound of formula (1), the compound having a saturated cycloalkyl group or a saturated heterocyclic group can also be produced by reduction of aryl and/or heteroaryl. The reaction can be carried out by selecting and using the reaction conditions described, for example, in "The fourth edition of Courses in Experimental Chemistry (Vol. 26)" edited by The Chemical Society of Japan, Maruzen, 1992. In addition, reduction of halogen(s) on the aryl and/or heteroaryl ring may be carried out using the reaction conditions described in Synthesis 1982, 10, 876-878.

(3) Amidation and Esterification

Among the compound of formula (1), the compound having an amide group or an ester group can be produced by reacting a compound having an amino group or a hydroxy group as a starting material with a carboxylic acid or a reactive derivative thereof. The reaction can be carried out referring, for example, to the methods described in "The fourth edition of Courses in Experimental Chemistry (Vol 22)" edited by The Chemical Society of Japan, Maruzen, 1992 and "The fifth edition of Courses in Experimental Chemistry (Vol. 16)" edited by The Chemical Society of Japan, Maruzen, 2005; or "'Compendium of Organic Synthetic Methods", Vols. 1 to 3, or the like.

(Production process of starting compound)

Of the starting compounds for Production Process 1 above, the starting compound (III) can be produced by the coupling of the indole ring to the benzene ring, for example in accordance with a method described in Journal of the American Chemical Society 2001, 123, 7727-7729.

Further, in the production of the starting compounds in Production Processes 2 to 5 above, an amino group of the starting compound can be prepared by reduction of a nitro group. The reaction can be carried out referring to the methods described in "The fourth eddition of Courses in Experimental Chemistry (Vol. 26)" edited by The Chemical Society of Japan, Maruzen, 1992.

(Test Examples)

An excellent selective inhibitory activity against human 17βHSD type 5 of the compound of formula (I) which can be used in the screening method of the present invention was confirmed by the test method described below. In this connection, the test may be carried out by referring to the details of test procedure described in Maniatis, T. et al., Molecular Cloning-A Laboratory Manual Cold Spring Harbor Laboratory, NY (1982) and the like. In addition, genes encoding human 17βHSD type$^5$ and type3 described in Sections 1 and 2 below, and human 17βHSD type 5 and type 3 may be obtained by the method described in Molecular Endocrinology, 1997, 11(13), 1971-1984.

Test Example 1. Isolation of gene encoding human 17βHSD type 5 and enzyme purification

A full-length cDNA encoding human 17βHSD type 5 used in the pharmacological test of this test example was obtained by the PCR. method using a cDNA derived from a human lung cancer-derived cell line, A549 cells as a template. The nucleotide sequence of the obtained cDNA was analyzed by the dideoxyterminator method, and the clone matched with the known human 17βHSD type 5 sequence (GenBank accession No. NM_003739) was selected. *Escherichia coli* BL21 was transformed with a plasmid containing the cDNA and cultured on a large scale. The proteins were purified by using GSTrapFF column (manufactured by Amersham) and PreScissionProtease (manufactured by Amersham). The purification method was carried out in accordance with the instructions attached to the GSTrapFF column.

Test Example 2. Isolation of gene encoding human 17βHSD type 3 and enzyme purification

A full-length cDNA encoding human 17βHSD type 3 used in the pharmacological test of this test example was obtained by the PCR method using a cDNA derived from human testis as a template. The nucleotide sequence of the obtained cDNA was analyzed by the dideoxyterminator method, and the clone matched with the known human 17βHSD type 3 sequence (GenBank accession No. BC034281) was selected. Subsequently, human fetus kidney-derived cell line, 293 cells, were transformed with a plasmid containing the cDNA, and the cells were collected 24 hours later. The collected cells were then disrupted in a phosphate buffer solution containing 5% glycerol (500 μL per 100 mm-dish of a phosphate buffer solution (pH 7.4, 200 mM) containing 5% glycerol) and centrifuged (16000 rpm, 5 min, 4°C), and the supernatant was used as an enzyme source.

Test Example 3. Measurement of enzyme activities of human 17βHSD type 5 and type 3

Enzyme activity was measured referring to Trevor M. Penning, et al., Biochem. J., 351, 67-77, (2000). Specifically, using a 100 mM potassium phosphate buffer (pH 6.0), (1) the enzyme purified in Section 1 at a final concentration of 10 μg/mL, (2) androstenedione at a final concentration of 300 nM, (3) NADPH at a final concentration of 200 μM, and (4) a test substance, were mixed to react at room temperature for 2 hours, and then the amount of testosterone produced was measured using DELFIA (trademark) Testosterone Reagents R050-201 (manufactured by PerkinElmer). The measurement was performed in accordance with the attached instructions. The amount of reduction of the testosterone production in the presence of the compound was obtained as a relative value with respect to the amount of testosterone in the absence of the enzyme set at 0% and the amount of testosterone produced in the absence of the compound set at 100%. Then, $IC_{50}$ values were calculated by the Logistic regression method.

Subsequently, UNCaP cells expressing human 17βHSD type 5 were constructed from a human prostate cancer-derived cell line, LNCaP cells, and a cell growth inhibitory activity of a Reference Example compound was evaluated.

Test Example 4. Construction of LNCaP cells expressing human 17βHSD) type 5

A full-length cDNA encoding human 17βHSD type 5 used in the pharmacological test of this test example was obtained by the PCR method using a cDNA derived from a human lung cancer-derived cell line, A549 cells, as a template. The nucleotide sequence of the obtained cDNA was analyzed by the dideoxyterminator method, and the clone matched with the known human 17βHSD type 5 sequence (GenBank accession No. NM_003739) was selected. The human prostate cancer-derived cell line LNCaP cells were transformed with a plasmid containing the cDNA and the cell line showing stable expression was obtained.

Test Example 5. Measurement of cell growth capability using LNCaP cells expressing human 17βHSD type 5

9000 cells/wel! of the transformed cells obtained in Section 4 above were seeded in a 96-well plate and cultured overnight. Then, androstenedione, in conjunction with a test compound, was added thereto at a final concentration of 10 nM, followed by culturing for 7 days. After the culturing, numbers of the cells were counted using a CellTiter-Glo (trademark) Luminescent Cell Viability Assay (Promega). The CellTiter-Glo (trademark) Luminescent Cell Viability Assay is a reagent that measures the number of the cells by monitoring an intracellular ATP level from the luminescence intensity by luciferase. The experimental manipulation was carried out in accordance with the attached instructions. The cell growth inhibitory activity in the presence of a test compound was calculated as a relative value with respect to the number of the cells in the absence of androstenedione set at proliferation of 0%, and the number of the cells in the presence of androstenedione and in the absence of the test compound set at proliferation of 100%. Then, $IC_{50}$ values were calculated by the Logistic regression method.

Table 1 shows the $IC_{50}$ values of inhibitory activity against human 17βHSD type 5 and type 3 and the $IC_{50}$ values of cell growth inhibitory activity using human 17βHSD type 5-expressing LNCaP cells of some Reference Example compounds. Abbreviation "Ex" represents Reference Example compound number.

[Table 1]

| Ex | Type5 $IC_{50}$ (nM) | Type3 $IC_{50}$ (nM) | LNCaP-17 β 5 $IC_{50}$(nM) |
|---|---|---|---|
| 1 | 200 | >10,000 | 6,677 |
| 6 | 50 | 1,800 | 46 |
| 17 | 180 | 6,400 | 66 |
| 20 | 120 | 7,300 | 101 |
| 21 | 110 | 4,700 | 102 |
| 32 | 120 | >10,000 | 46 |
| 38 | 96 | >10,000 | 45 |
| 44 | 40 | >10,000 | 34 |
| 47 | 180 | 2,500 | 92 |
| 54 | 70 | 3,200 | 141 |

As shown by the test results above, the compounds of formula (I) hardly have an inhibitory activity against human 17βHSD type 3 and have an inhibitory activity selective to human 17βHSD type 5.

Further, as shown by the test results above, since the compounds of formula (I) have very weak inhibitory activity against human 17βHSD type 3, they are expected to suppress intracrine testosterone synthesis selectively in the prostate by their selective inhibitory effects against 17βHSD type 5 without affecting biosynthesis of testosterone derived from human 17βHSD type 3 in the testes, thus useful for treating and/or preventing 17βHSD type 5-related diseases such as benign prostatic hyperplasia and prostate cancer without adverse effects.

Further, as shown by the test results above, since the compounds of formula (I) exhibited cell growth inhibitory activity in the human 17βHSD) type 5-expressing LNCaP cells and they suppressed the testosterone production in the tumor in the CWR22R tumor-bearing mouse in Examples below, they suppress intracrine testosterone synthesis selectively in prostate cancer by their selective inhibitory effects against 17βHSD type 5, thus can be used for treating and/or preventing prostate cancer without adverse effects.

A preparation containing one or two or more kinds of the compound of formula (1) or a salt thereof as an active ingredient can be prepared in accordance with a generally used method, using a pharmaceutical carrier, excipient, or the like, that is usually used in the art.

The administration can be carried out in any form of oral administration via tablets, pills, capsules, granules, powders, liquid preparations, or the like, or parenteral administration via injections such as intraarticular injection, intravenous injection, intramuscular injection, or the like, as well as suppositories, eye drops, eye ointments, percutaneous liquid preparations, ointments, percutaneous patches, transmucosal liquid preparations, transmucosal patches, inhalations, and the like.

As solid compositions for oral administration, tablets, powders, granules, or the like are used. In such a solid composition, one or two or more kinds of active ingredients are mixed with at least one inert excipient such as lactose, mannitol, glucose, hydroxypropylcellulose, microcrystalline cellulose, starch, polyvinyl pyrrolidone, and/or magnesium aluminometasilicate. According to a conventional method, the composition may contain inert additives such as a lubricant such as magnesium stearate, a disintegrator such as sodium carboxymethyl starch, a stabilizing agent, and a solubilizing agent. As occasion demands, the tablets or the pills may be coated with a sugar coating, or a film of gastric or enteric materials.

Liquid compositions for oral administration include pharmaceutically acceptable emulsions, solutions, suspensions, syrups, elixirs, or the like, and contain a generally used inert diluent such as purified water or ethanol. In addition to the inert, diluent, the liquid composition may contain an adjuvant such as a solubilizing agent, a moistening agent, and a suspending agent, a sweetener, a flavor, an aroma, and an antiseptic.

Injections for parenteral administration include sterile aqueous or non-aqueous solutions, suspensions and emulsions. The aqueous solvent includes, for example, distilled water for injection and physiological saline. Examples of the non-aqueous solvent include propylene glycol, polyethylene glycol, vegetable oils such as olive oil, alcohols such as ethanol, Polysorbate 80 (Japanese Pharmacopeia), and the like. Such a composition may further contain a tonicity agent, an antiseptic, a moistening agent, an emulsifying agent, a dispersing agent, a stabilizing agent, or a solubilizing agent. These are sterilized, for example, by filtration through a bacteria-retaining filter, blending of a sterilizing agent, or irradiation. In addition, these can also be used by preparing a sterile solid composition, and dissolving or suspending it in sterile water or a sterile solvent for injection prior to use.

External preparations include ointments, plasters, creams, jellies, cataplasms, sprays, lotions, eye drops, eye ointments, and the like. Generally used ointment bases, lotion bases, aqueous or non-aqueous liquids, suspensions, emulsions, and the like are included. Examples of the ointment or lotion bases include polyethylene glycol, propylene glycol, white Vaseline, bleached beewax, polyoxyethylene hydrogenated castor oil, glyceryl monostearate, stearyl alcohol, cetyl alcohol, lauromacrogol, sorbitan sesquioleate, and the like.

As the transmucosal preparations such as inhalations and transnasal preparations, a solid, liquid or semi-solid form are used, and can be prepared in accordance with a conventionally known method. For example, a known excipient, and also a pH-adjusting agent, an antiseptic, a surfactant, a lubricant, a stabilizing agent, a thickening agent, or the like may be appropriately added thereto. For their administration, an appropriate device for inhalation or blowing can be used. For example, a compound may be administered alone or as a powder of formulated mixture, or as a solution or suspension in combination with a pharmaceutically acceptable carrier, using a conventionally known device or sprayer, such as a measured administration inhalation device. The dry powder inhalation devices or the like may be for single or multiple administration use, and a dry powder or a powder-containing capsule can be used. Alternatively, it may be in a form such as a pressurized aerosol spray or the like which uses an appropriate propellant, for example, a suitable gas such as chlorofluoroalkane, hydrofluoroalkane, or carbon dioxide and the like.

In oral administration, the daily dose is generally from about 0.001 to 100 mg/kg, preferably from 0. to 30 mg/kg, and more preferably 0.1 to 10 mg/kg, per body weight, administered in one portion or in 2 to 4 divided portions. In the case of intravenous administration, the daily dose is suitably from about 0.0001 to 10 mg/kg per body weight, once a day or two or more times a day. In addition, a transmucosal agent is administered at a dose from about 0.001 to 100

mg/kg per body weight, once a day or two or more times a day. The dose is appropriately decided in response to the individual case by taking the symptoms, the age, and the gender, and the like into consideration.

The compounds of formula (I) can be used in combination with various agents for treating and/or preventing the diseases for which the compounds of formula (I) are considered to be effective. The combined preparation may be administered simultaneously, or separately and continuously or at a desired time interval. The preparations to be co-administered may be a blend, or may be prepared individually,

(Reference Examples)

The production processes for the compounf of formula (I) which can be used in the screening method of the present invention will be described below as Reference Examples. Production processes for novel compounds among the compounds used as starting materials of compounds of formula (I) will be described as Production Examples. The production processes for the compound of formula (I) are not limited to the production processes in specific Reference Examples shown below and can be produced by a. combination of these production precesses or known production processes.

The following abbreviations are used in Production Examples, Reference Examples and Tables below.
Ex: Reference Example number, REx: Production Example number. Data: physicochemical data (FAB+: FAB-MS $(M+H)^+$, FAB-; FAB-MS $(M-H)^-$, ESI+: ESI-MS $(M+H)^+$, ESI-: ESI-MS $(M-H)^-$, API+: API-ES-MS $(M+H)^+$, EI: EI-MS $(M)^+$ NMR-DMSOd6: δ(ppm) of peak(s) in $^1H$ NMR in DMSO-$d_6$), Str: Structural formula, Syn (REx): Production Example numbers in which the corresponding compounds were produced using the same method, Syn (Ex): Reference Example numbers in which the corresponding compounds were produced using the same method, DME: dimethoxyethane, DMF: N,N-dimethylformamide, DMSO: dimethyl sulfoxide. IHF: tetrahydrofuran, MeCN: acetonitrile, MeOH: methanol, tBuOH: tert-butyl alcohol, n-BuLi: n-butyllithium, RT: retention time (minutes) in HPLC.

Production Example 1

A mixture of 5.03 g of 2-fluoro-4-methoxy-1-nitrobenzene and 100 mL of THE was cooled to -50˚C, and 90 mL of 1M vinyl magnesium bromide-THF solution was added thereto at -30˚C or lower, followed by stirring at -50˚C for 2 hours. To the reaction solution were added 100 mL of a saturated aqueous ammonium chloride solution and 90 mL of 1M hydrochloric acid, followed by warming to room temperature and stirring for 15 minutes. The mixture was extracted twice with 100 mL of ethyl acetate. The extract was washed with water and saturated brine and dried over anhydrous sodium sulfate, and then the solvent was evaporated under reduced pressure. The residue was purified by silica gel column chromatography (ethyl acetate-hexane=1:20→1:9) to obtain 470 mg of 7-fluoro-5-methoxy-1H-indole as a brown oily substance.

Production Example 2

To a mixture of 1.35 g of ethyl 3-phenyl-5-(trifluoromethyl)-1H-indole-2-carboxylate, 10 mL of methanol and 10 mL of THF was added 6.0 mL of a 1M aqueous sodium hydroxide solution, followed by stirring at 50˚C for 3 hours. 30 mL of water and 6 mL of 1M hydrochloric acid were added to the reaction solution, and the precipitated solid was collected by filtration and dried. The solid obtained was dissolved in 15 mL of N-methyl-2-pyrrohdinone, and 100 mg of copper powder was added thereto, followed by stirring at 160˚C overnight. The reaction solution was allowed to cool, and 100 mL of ethyl acetate was added thereto to remove insoluble materials, followed by washing with water and saturated brine and drying over anhydrous sodium sulfate, and then the solvent was evaporated under reduced pressure. The residue was purified by silica gel column chromatography (ethyl acetate-hexane=1:4) to obtain 770 mg of 3-phenyl-5-(trifltioromethyl)-1H-indole as a brown oily substance.

Production Example 3

A mixture of 120 mg of copper (I) iodide, 160 mg oftrans-N,N'-dimethylcyclohexane-1,2-diamine and 6 mL of dioxane were added with 1.2 mL of ethyl 3-iodobenzoate, 850 mg of 1H-indole-5-carbomtrile and 1.65 g of potassium carbonate, followed by stirring at 110˚C overnight. The reaction solution was allowed to cool, 50 mL of ethyl acetate was added thereto to remove insoluble materials, and the solvent was evaporated under reduced pressure. The residue was purified by silica gel column chromatography (eluent: chloroform), and washed with ether-hexane (1:5) to obtain 1.41 g of ethyl 3-(5-cyano-1H-indol-1-yl)benzoate as a white solid.
Compounds of Production Examples 4 to 10 listed in Tables 2 and 3 were obtained in the same manner as in Production Example 3.

Production Example 11

A mixture of 1.0 g of 4-fluoro-3-nitrobenzonitrile, 1.09 g of ethyl 3-aminobenzoate, 1.25 g of potassium carbonate and 10 mL of DMF was stirred at 100˚C for 3 hours. 60 mL of water was added to the reaction solution. The mixture was extracted with 120 mL of ethyl acetate. The extract was washed with water and saturated brine and dried over anhydrous sodium sulfate, and then the solvent was evaporated under reduced pressure. The residue was washed with ethanol to obtain 1.43 g of ethyl 3-[(4-cyano-2-nitropbenyl)ammo]benzoate as a brown solid,
A Compound of Production Example 12 listed in Table 3 was obtained in the same manner as in Production Example 11.

Production Example 13

To a mixture of 85 mg of palladium acetate, 350 mg of 2,2'-bis(diphenylphosphino)-1,1'-binaphthyl and 15 mL of toluene were added 1.8 mL of ethyl 3-bromobenzoate, 1.02 g of 2-nitroaniline and 4.81 g of cesium carbonate, followed by stirring at 110˚C for 5 hours. The reaction solution was allowed to cool, 50 mL of ethyl acetate was added thereto to remove insoluble materials, and the solvent was evaporated under reduced pressure. The residue was purified by silica gel column chromatography (eluent: ethyl acetate-hexane=1:9) to obtain 2.10 g of ethyl 3-[(2-nitrophcnyl)ammo]benzoate as an light orange oily substance.
Compounds of Production Examples 14 to 16 listed in Table 3 were obtained in the same manner as in Production Example 13.

Production Example 17

To a mixture of 1.42 g of ethyl 3-[(4-cyano-2-nitrophenyl)amino]benzoate and 10 mL of acetic acid was added 0.77 g of iron powder, followed by stirring at 80˚C for 2 hours. Insoluble materials were removed from the reaction solution, and the solvent was evaporated under reduced pressure. 100 mL of a saturated aqueous sodium hydrogen carbonate solution was added to the residue, followed by extraction with 50 mL of chloroform twice. The extract was dried over anhydrous sodium sulfate, and then the solvent was evaporated under reduced pressure to obtain 1.35 g of ethyl 3-[(2-ammo-4-cyanophenyl)amino]benzoate as a pale yellow solid.
Compounds of Production Examples 18 to 20, 76 and 77 listed in Tables 4 and 13 were obtained in the same manner as in Production Example 17.

Production Example 21

To a mixture of 400 mg of ethyl 3-[(2-amino-4-cyanophenyl)amino]benzoate and 5 mL of THF was added 0.12 mL of acetyl chloride, followed by stirring at room temperature for 2 hours and at 70˚C for 2 hours. The reaction solution was allowed to cool, and 30 mL of a saturated aqueous sodium hydrogen carbonate solution was added thereto, followed by extraction with 60 mL of ethyl acetate, The extract was washed with water and saturated brine and dried over anhydrous sodium sulfate, and then the solvent was evaporated under reduced pressure. The residue was purified by silica gel column chromatography (eluent: ethyl acetate-hexane=1:1) to obtain 360 mg of ethyl 3-(5-cyano-2-methyl 1H-benzimidazol-1-yl)benzoate as a white solid.
Compounds of Production Examples 22 to 48 listed in Tables 4 to 8 were obtained in the same manner as in Production Example 21.

Production Example 49

To a solution of 315 mg of ethyl 3-[(2-amino-4-cyanophenyl)amino]benzoate in 6.3 mL of THF was added 232 mg of (3-fluorophenyl) acetyl chloride, followed by stirring at room temperature for 2.5 hours, and at 80˚C for 16 hours. The reaction solution was allowed to cool, and 20 mL of a saturated aqueous sodium hydrogen carbonate solution was added thereto, followed by extraction with 50 mL of ethyl acetate. The extract was washed with water and saturated brine and dried over anhydrous magnesium sulfate, and then solvent was evaporated under reduced pressure. The resulting residue was purified by silica gel column chromatography (eluent: ethyl acetate-hexane=1:6) to obtain 189 mg of ethyl 3-[5-cyano-2-(3-fluorobenzyl)-1H-benzimidazol-1-yl]benzoate as a colorless oily substance.
Compounds of Production Examples 50 to 55 listed in Tables 9 and 10 were obtained in the same manner as in Production Example 49.

Production Example 56

To a mixture of 320 mg of ethyl 3-{[2-amino-4-(trifluoromethyl)phenyl]amino} benzoate and 4 mL of ethanol

was added 310 mg of formamidine acetate, followed by stirring under heating to reflux overnight. The reaction solution was allowed to cool, and 30 mL of a saturated aqueous sodium hydrogen carbonate solution was added thereto, followed by extraction with 60 mL of ethyl acetate. The extract was washed with water and saturated brine and dried over anhydrous sodium sulfate, and then the solvent, was evaporated under reduced pressure. The residue was purified by silica gel column chromatography (eluent: chloroform) and washed with hexane to obtain 290 mg of ethyl 3-[5-(trifluoromethyl)-1H-benzimidazol-1-yl]benzoate as a pale yellow solid.
A compound of Production Example 57 listed in Table 10 was obtained in the same manner as in Production Example 56.

Production Example 58

To a solution of 545 mg of ethyl 3-{[2-amino-4-(trifluoromethyl)phenyl]amino} benzoate in 10 mL of acetonitrile were added 0.468 mL of triethylamine and 409 mg of 1,1-carbonvibis (1H-imiidazole), followed by stirring at 90˚C for 7 hours. After being cooled to room temperature, the resulting precipitate was collected by filtration to obtain 499 mg of ethyl 3-[2-oxo-5-(trifluoromethyl)-2,3-dihydro-1H-benzimidazol-1-yl]benzoate as a white solid.

Production Example 59

To a solution of 889 mg of ethyl 3-[2-oxo-5-(trifluoromethyl)-2,3-dihydro-1H-benzimidazol-1-yl]benzoate in 10 mL of phosphorus oxychloride was added 793 mg of phosphorus pentachloride, followed by stirring at 100˚C for 15 hours. After being cooled to room temperature, the reaction solution was concentrated under reduced pressure. Ethyl acetate and saturated aqueous sodium bicarbonate were added to the resulting residue, and the organic layer was separated and washed with saturated brine. The organic layer was dried over anhydrous sodium sulfate, and then the solvent was evaporated under reduced pressure to obtain 985 mg of ethyl 3-[2-chloro-5-(trifluoromethyl)-1H-benzimidazol-1-yl]benzoate as a pale yellow oily substance.

Production Example 60

To a solution of 588 mg of ethyl 3-[2-chloro-5-(trifluoromethyl)-1H-benzimidazol-1-yl]benzoate in 5 mL of methanol was added 0.920 mL of 28% sodium methoxide methanol solution, followed by stirring at room temperature for 16 hours. The resulting precipitate in the reaction solution was collected by filtration to obtain 384 mg of methyl 3-[2-methoxy-5-(trifluoromethyl)-1H-benzimidazol-1-yl]benzoate as a white solid.

Production Example 61

To a solution of 111 mg of ethyl 3-[2-chloro-5-(trifluoromethyl)-1H-benzimidazol-1-yl]benzoate in 3 mL of THF was added 0.330 mL of a 1M aqueous sodium hydroxide solution, followed by stirring at room temperature for 15 hours. 0.330 mL of a 1M aqueous hydrochloric acid solution was added under ice cooling to the reaction solution, and ethyl acetate and saturated brine were added thereto, followed by separation of the organic layer. The organic layer was dried over anhydrous sodium sulfate, and then the solvent was evaporated under reduced pressure to obtain 113 mg of 3-[2-chloro-5-(trifluoromethyl)-1H-benzimidazol-1-yl]benzoic acid as a white solid.

Production Example 62

To a mixture of 1.15 g of ethyl 3-[2-pyridin-4-yl-5-(trifluoromethyl)-1H-benzimidazol-1-yl]benzoate, 0.5 mL of concentrated hydrochloric acid and 15 mL of ethanol was added 0.15 g of platinum (IV) oxide, followed by stirring overnight under a hydrogen atmosphere at room temperature. The reaction mixture was filtered and then the filtrate was concentrated under reduced pressure. 60 mL of ethyl acetate and 30 mL of a saturated aqueous sodium hydrogen carbonate solution were added to the residue, and the organic layer was separated. The organic layer was washed with water and saturated brine and dried over anhydrous sodium sulfate, and then the solvent was evaporated under reduced pressure. The residue was purified by silica gel column chromatography (eluent: chloroform-methanol-concentrated aqueous ammonia=20:1:0.1→10:1:0.1) to obtain 1.05 g of ethyl 3-[2-piperidin-4-yl--5-(trifluoromethyl)-1H-benzimidazol-1-yl]benzoate as a white amorphous solid.

Production Example 63

To a mixture of 420 mg of ethyl 3-[2-piperidin-4-yl-5-(trifluoromethyl)-1H-benzimidazol-1-yl]benzoate, 0.17 mL of triethylamine and 4 mL of THF was added 0.12 mL of acetic anhydride, followed by stirring at room temperature for 5 hours. 60 mL of ethyl acetate was added to the reaction solution. The mixture was washed with water and saturated

brine and dried over anhydrous sodium sulfate, and the solvent was evaporated under reduced pressure. The residue was purified by silica gel column chromatography (eluent: chloroform-methanol=100:1) to obtain 430 mg of ethyl 3-(2-(1-acetylpiperidin-4-yl)-5-(trifluoromethyl)-1H-benzimidazol-1-yl]benzoate as a white amorphous solid.

Production Example 64

272 mg of ethyl 3-[2-(4-bromobenzyl)-5-(trifluoromethyl)-1H-benzimidazol-1-yl]benzoate was dissolved in 2.7 mL of ethanol, and 13.6 mg of palladium-carbon was added thereto, followed by stirring under a hydrogen atmosphere at room temperature for 3 hours. The reaction mixture was filtered, and the filtrate was concentrated under reduced pressure. The resulting residue was dissolved in ethyl acetate, and washed with aqueous sodium bicarbonate, water and saturated brine. This solution was dried over anhydrous magnesium sulfate, and the solvent was evaporated under reduced pressure. The resulting residue was purified by silica gel column chromatography (eluent: ethyl acetate-hexane=1:6) to obtain 229 mg of ethyl 3-[2-benzyl-5-(trifluoromethyl)-1H-benzimidazol-1-yl]benzoate as a pale violet oily substance.

Production Example 65

To a mixture of 300 mg of ethyl 3-{[2-amino-4-(trifluoromethyl)phenyl]amino} benzoate, 0.11 mL of pyridine and 3 mL ofTHF was added 0.16 mL of cyclohexanecarbonyl chloride, followed by stirring at room temperature for 1 hour, and at 80˚C for 5 hours. The reaction solution was allowed to cool, 30 mL of water was added thereto, and the precipitated solid was collected by filtration. The solid obtained was washed with hexane to obtain 360 mg of ethyl 3-({2-[(cyclohexylcarbonyl)amino]-4-(trifluoromethyl)phenyl}amino)benzoate as a pale yellow solid.
Compounds of Production Examples 66 to 70 listed in Tables 11 and 12 were obtained in the same manner as in Production Example 65.

Production Example 71

To a mixture of 340 mg of ethyl 3-({2-[(cyclohexy1carbonyl)amino]-4-(trifluoromethyl)phenyl}amino)benzoate, 2.5 mL of methanol and 2.5 mL of THF was added 1.1 mL of a 1M aqueous sodium hydroxide solution, followed by stirring at 50˚C for 3 hours. 1.1 mL of 1M hydrochloric acid and 30 mL of water were added to the reaction solution, and the precipitated solid was collected by filtration. The solid obtained was washed with ethanol to obtain 210 mg of 3-({2-[(cyclohexy1carbonyl)amino]-4-(trif1uoromethyl)phenyl}amino)benzoic acid as a white solid.
Compounds of Production Examples 72 to 74, 78 and 79 listed in Tables 12 and 13 were obtained in the same manner as in Production Example 71.

Production Example 75

To a mixture of 324 mg of ethyl 3-{[2-amino-4-(trifluoromethyl)phenyl]amino} benzoate in 5 mL of a mixture of ethanol and THF was added 1.50 mL of a 1M aqueous sodium hydroxide solution, followed by stirring at room temperature for 2 hours. 1.00 mL of 1 M aqueous sodium hydroxide solution was added to the reaction solution, followed by stirring at room temperature for 27 hours. The reaction solution was concentrated under reduced pressure, and 2.50 mL of 1M aqueous hydrochloric acid solution and 10 mL of water were added thereto under ice cooling to the resulting residue. The resulting precipitate was collected by filtration to obtain 281 mg of 3-([2-amino-4-(trifluoromethyl)phenyl]mllino} benzoic acid as a brown solid.
The structural formula, production process and physicochemical data of each of th e Production Example compounds are shown in the tables.
[Table 2]

| REx | Syn (REx) | Str | Data |
|---|---|---|---|
| 1 | 1 | 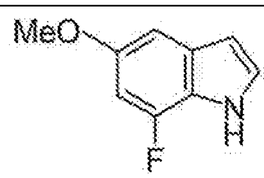 | EI: 165 |

(continued)

| REx | Syn (REx) | Str | Data |
|---|---|---|---|
| 2 | 2 | 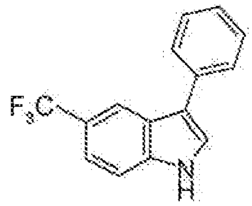 | EI: 261 |
| 3 | 3 | 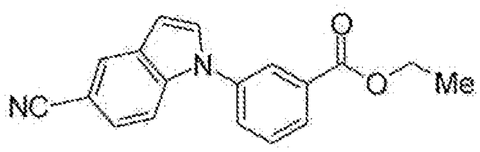 | FAB+: 291 |
| 4 | 3 | 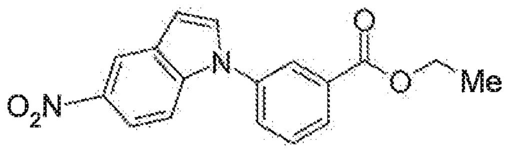 | FAR+: 311 |
| 5 | 3 | 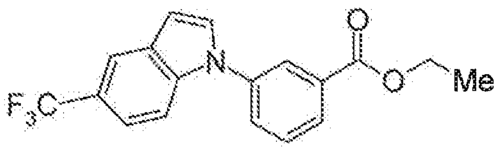 | FAB+: 334 |
| 6 | 3 | 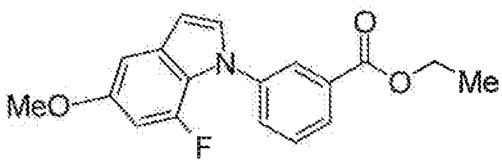 | EI: 313 |
| 7 | 3 | 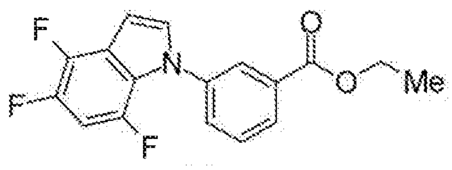 | EI: 319 |
| 8 | 3 | 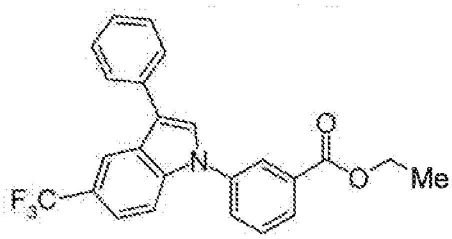 | FAB+: 410 |

[Table 3]

| REx | Syn (REx) | Str | Data |
|---|---|---|---|
| 9 | 3 | 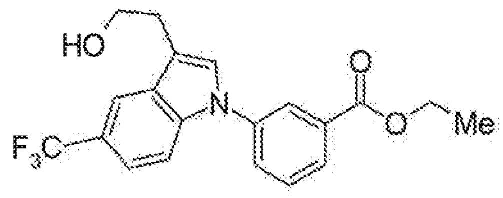 | FAB+: 378 |
| 10 | 3 | 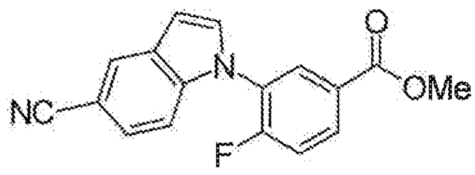 | CI+: 295 |
| 11 | 11 | 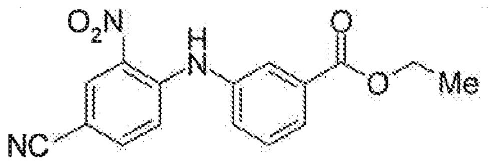 | ESI: 310 |
| 12 | 11 | 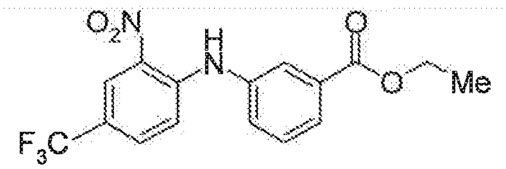 | FAB-: 353 |
| 13 | 13 | 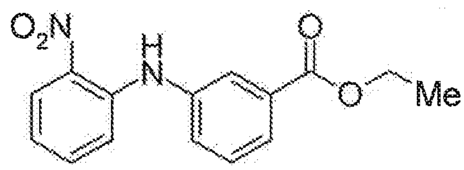 | FAB-: 286 |
| 14 | 13 | 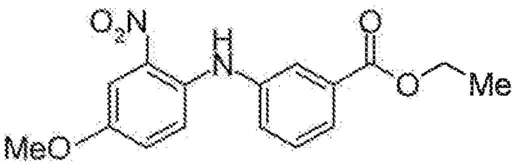 | FAB-: 315 |
| 15 | 13 | 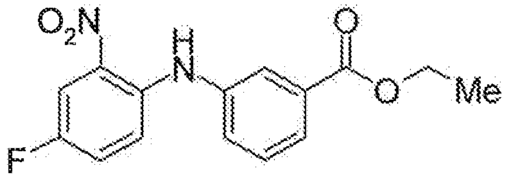 | FAB-: 304 |
| 16 | 13 | 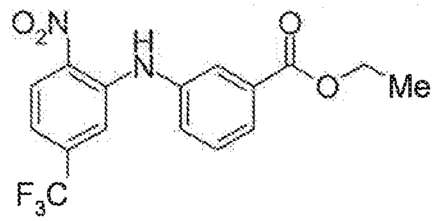 | FAB+: 354 |

[Table 4]

| REx | Syn (REx) | Str | Data |
|---|---|---|---|
| 17 | 17 | 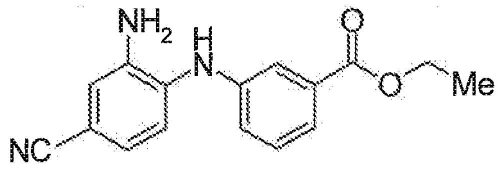 | FAB+: 282 |
| 18 | 17 | 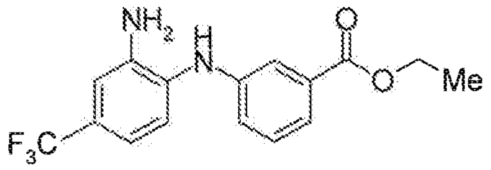 | Fay-: 323 |
| 19 | 17 | 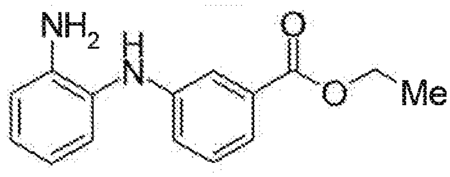 | EI: 256 |
| 20 | 17 | 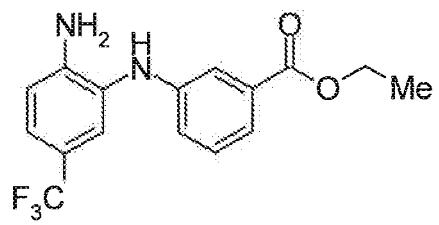 | EI: 324 |
| 21 | 21 | 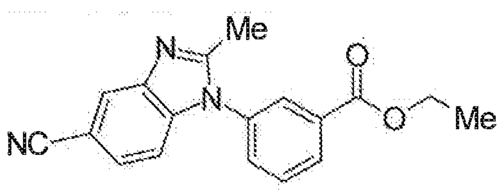 | FAB+: 306 |
| 22 | 21 | 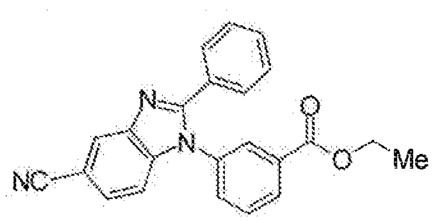 | ESI+:368 |
| 23 | 21 | 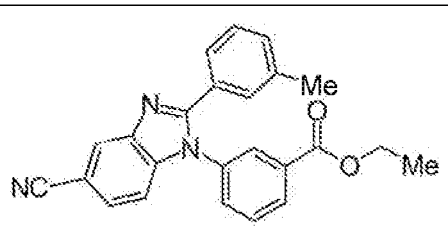 | FAB+: 382 |

[Table 5]

| REx | Syn (REx) | Str | Data |
|---|---|---|---|
| 24 | 21 | 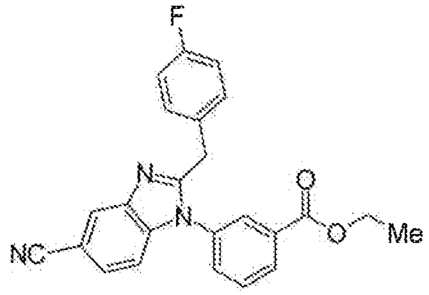 | ESI+: 400 |
| 25 | 21 | 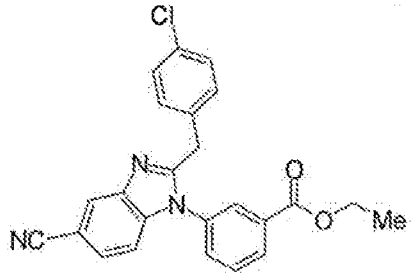 | ESI+: 416 |
| 26 | 21 | 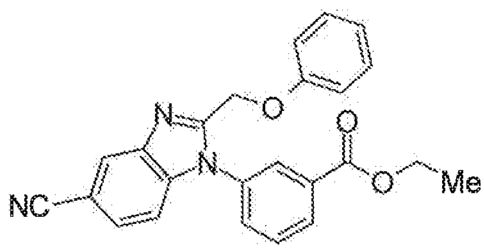 | ESI+: 398 |
| 27 | 21 | 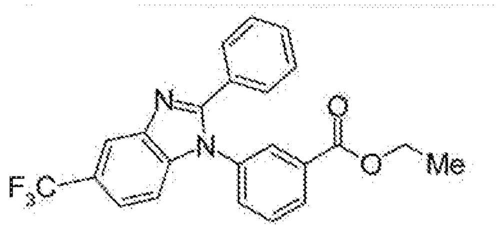 | ESI+: 411 |
| 28 | 21 | 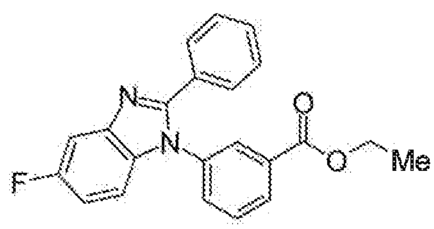 | FAB+: 361 |
| 29 | 21 | 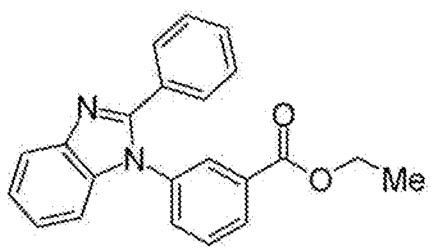 | EI: 342 |

[Table 6]

| REx | Syn (REx) | Str | Data |
|---|---|---|---|
| 30 | 1 | 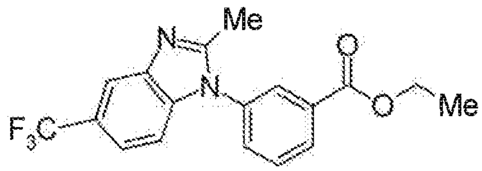 | ESI+: 349 |
| 131 | 2 | 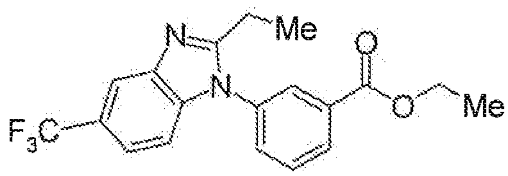 | FAB+: 363 |
| 32 | 21 | 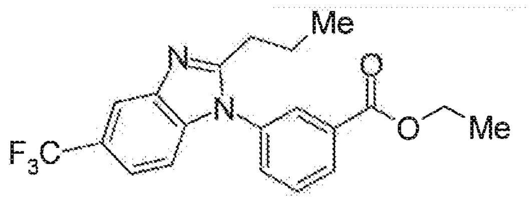 | FAB+: 377 |
| 33 | 21 | 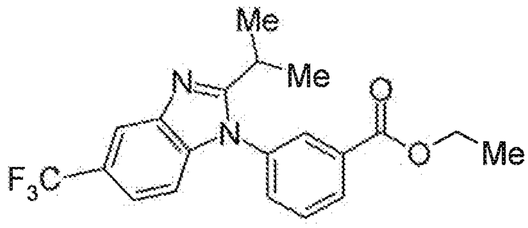 | FAB+: 377 |
| 34 | 21 | 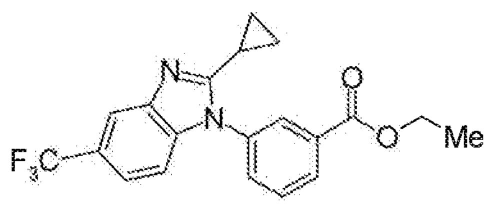 | FAB+: 375 |
| 35 | 21 | 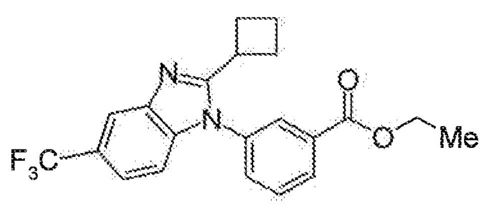 | FAB+: 389 |
| 36 | 21 | 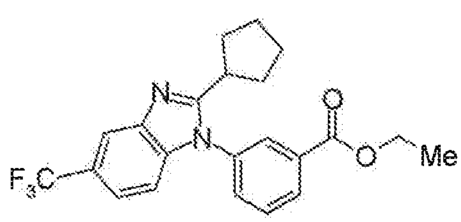 | FAB+: 403 |

[Table 7]

| REx | Syn (REx) | Str | Data |
|---|---|---|---|
| 37 | 21 | 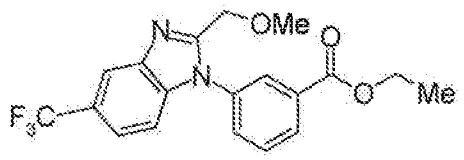 | FAB+: 379 |
| 38 | 21 | 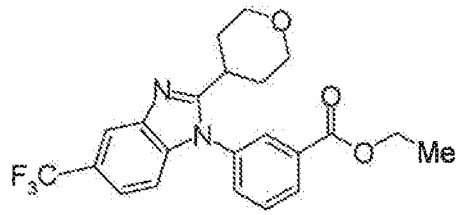 | FAS+: 419 |
| 39 | 21 | 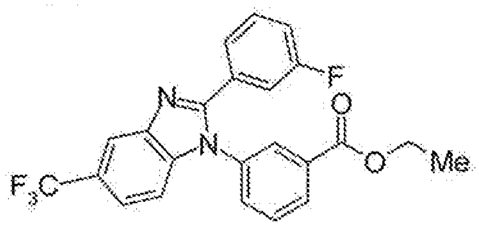 | FAB+: 429 |
| 40 | 21 | 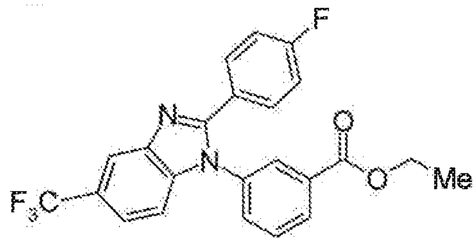 | FAB+: 429 |
| 41 | 21 | 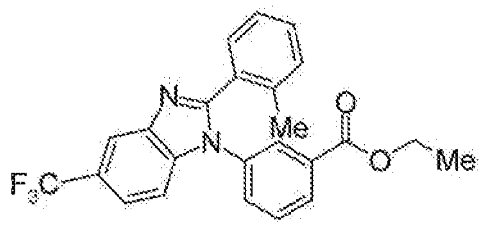 | FAB+: 425 |
| 42 | 21 | 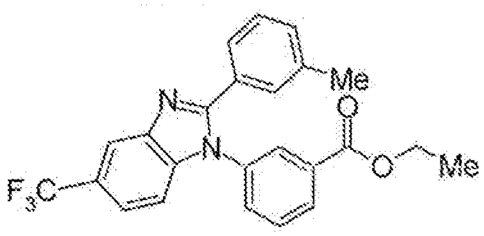 | FAB+: 425 |

[Table 8]

| REx | Syn (REx) | Str | Data |
|---|---|---|---|
| 43 | 21 | 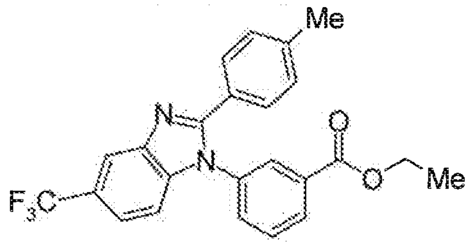 | FAB+: 425 |
| 44 | 21 | 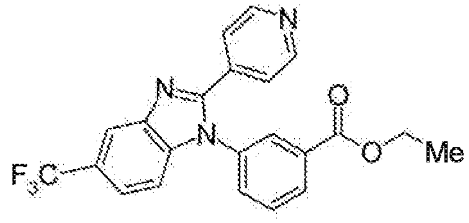 | FAB+: 412 |
| 45 | 21 | 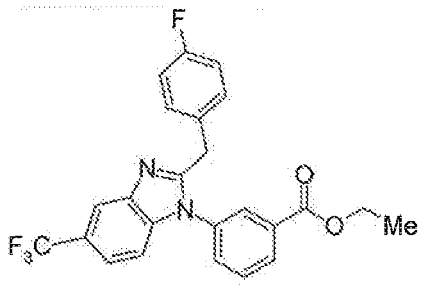 | ESI+ 443 |
| 46 | 21 | 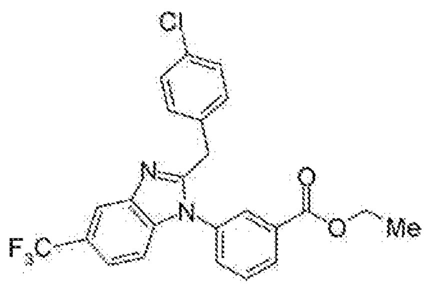 | ESI+:459 |
| 47 | 21 | 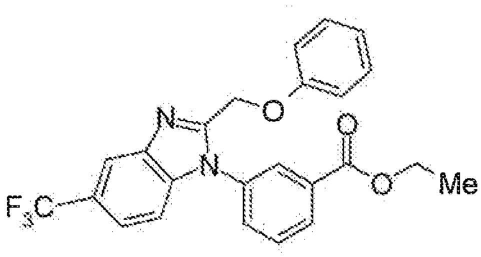 | FAB+: 441 |
| 48 | 21 | 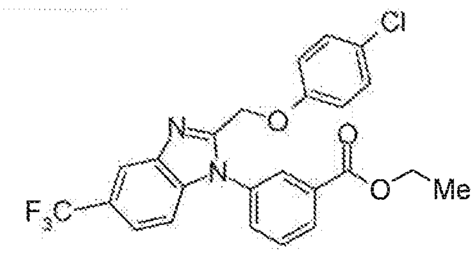 | FAB+: 475 |

[Table 9]

| REx | Syn (REx) | Str | Data |
|---|---|---|---|
| 49 | 49 | 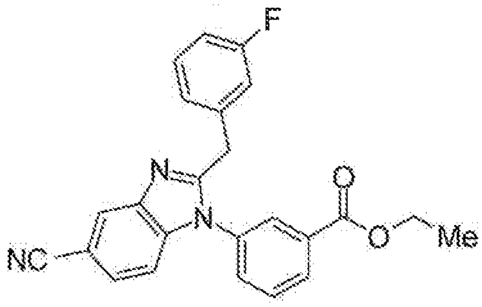 | ESI+: 400 |
| 50 | 49 | 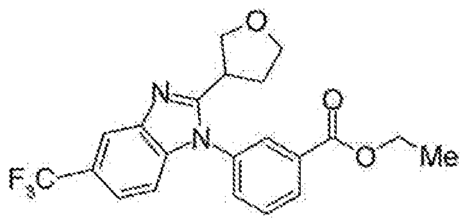 | FAB+: 405 |
| 51 | 49 | 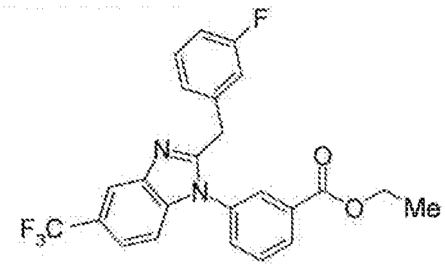 | ESI+: 443 |
| 52 | 49 | 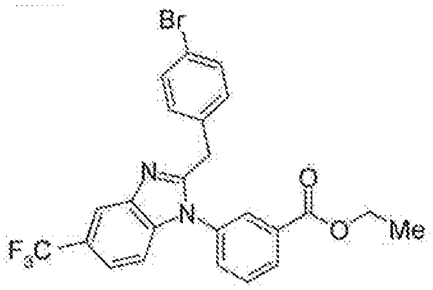 | ESI+: 502 |
| 53 | 49 | 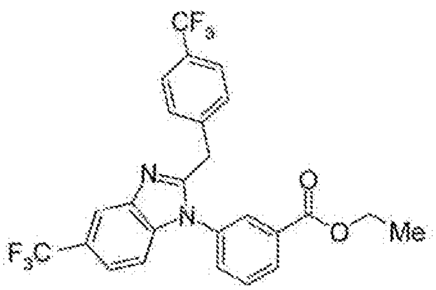 | ESI+: 494 |

[Table 10]

| REx | Syn (REx) | Str | Data |
|---|---|---|---|
| 54 | 49 | 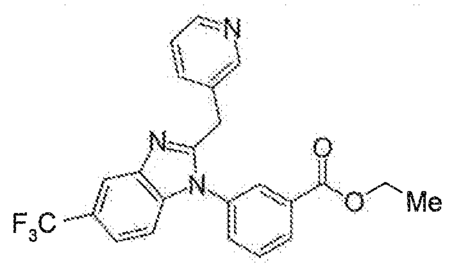 | ESI+:426 |
| 55 | 49 | 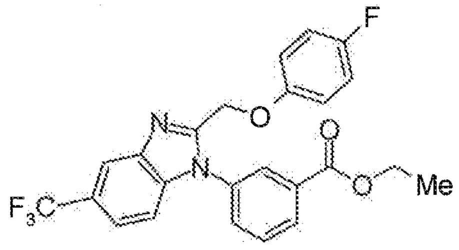 | FAB+: 459 |
| 56 | 49 | 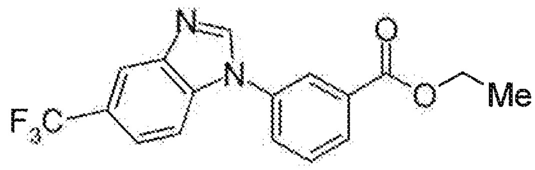 | FAB+: 335 |
| 57 | 56 | 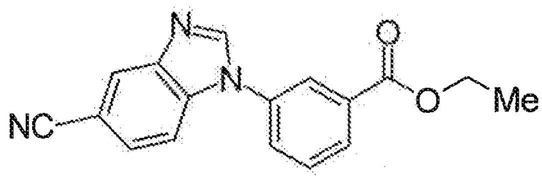 | FAB+: 292 |
| 58 | 58 | 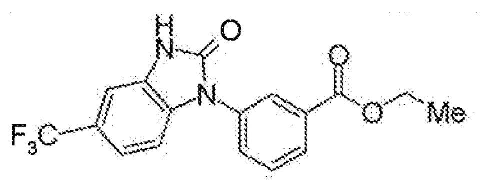 | FAB 351 |
| 59 | 59 | 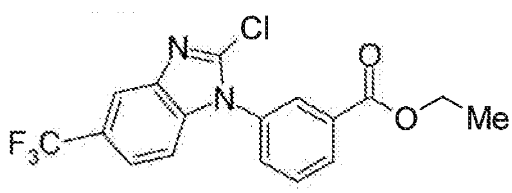 | FAB+: 369 |
| 60 | 60 | 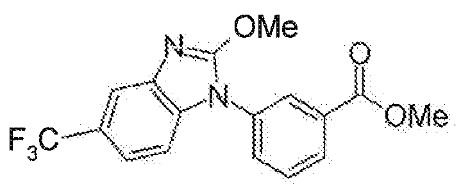 | FAB+: 351 |

[Table 11]

| REx | Syn (Rex) | Str | Data |
|---|---|---|---|
| 61 | 61 | 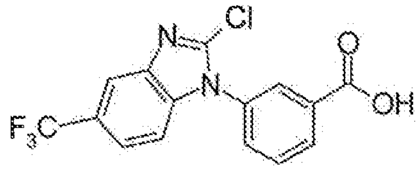 | FAB+: 341 |
| 62 | 62 | 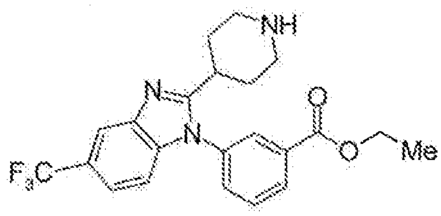 | FAB+: 418 |
| 63 | 63 | 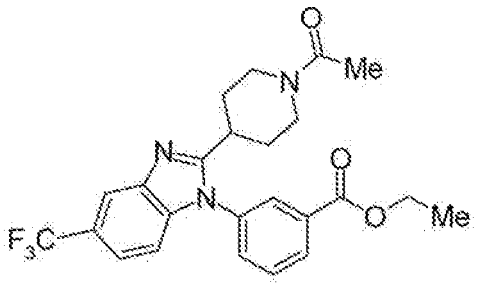 | FAB+: 460 |
| 64 | 64 | 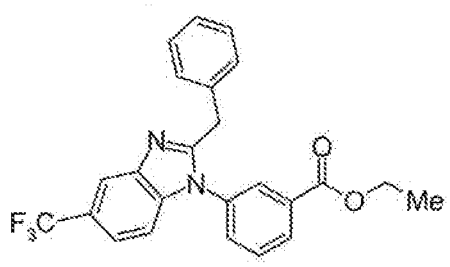 | API+: 425 |
| 65 | 65 | 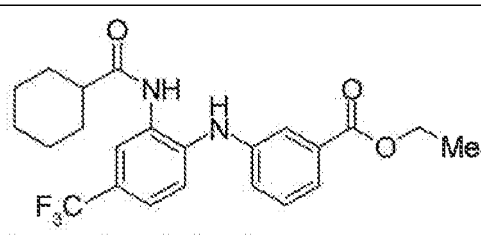 | FAB+: 435 |
| 66 | 65 | 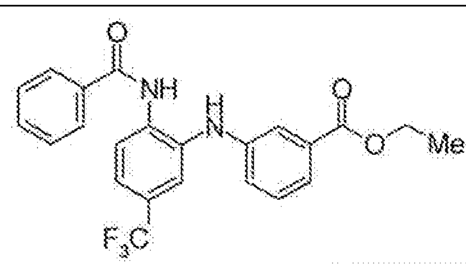 | FAB+: 429 |

[Table 12]

| REx | Syn (REx) | Str | Data |
|---|---|---|---|
| 67 | 65 | 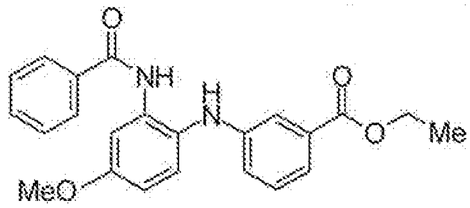 | FAB+: 391 |
| 68 | 65 | 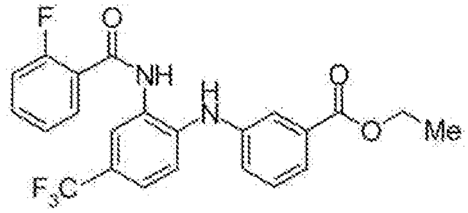 | FAB+: 447 |
| 69 | 65 | 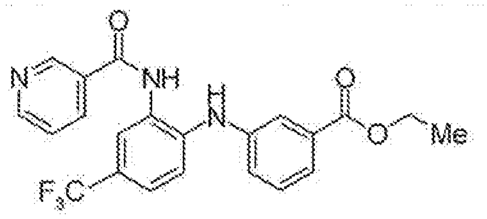 | FAB+: 430 |
| 70 | 65 | 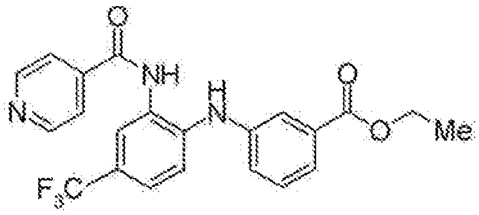 | FAB+: 430 |
| 71 | 71 | 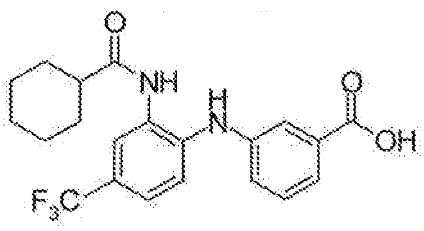 | FAB-: 405 |
| 72 | 71 | 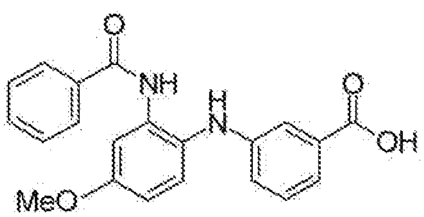 | ESI+: 363 |

[Table 13]

| REx | Syn(REx) | Str | Data |
|---|---|---|---|
| 73 | 71 | 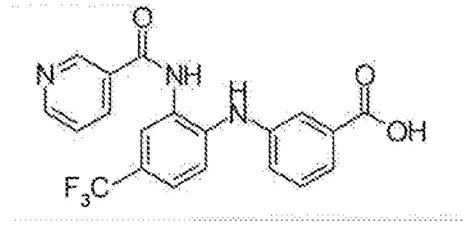 | ESI-: 400 |
| 74 | 71 | 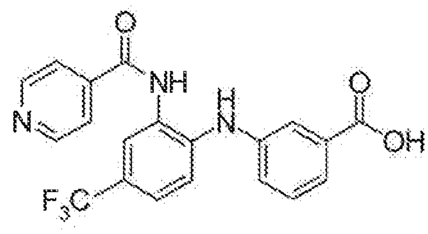 | FAB+: 402 |
| 75 | 75 | 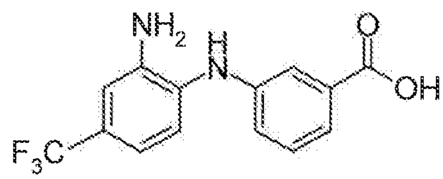 | ESI-: 295 |
| 76 | 17 | 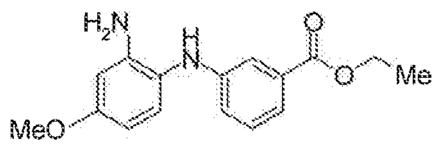 | |
| 77 | 17 | 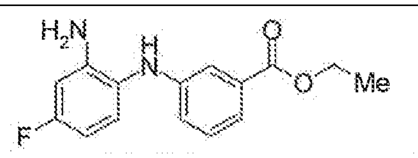 | |
| 78 | 71 | 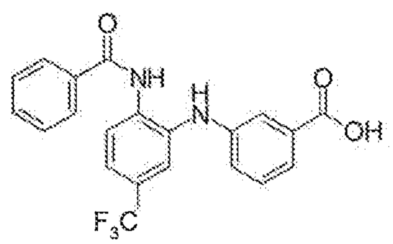 | |
| 79 | 71 | 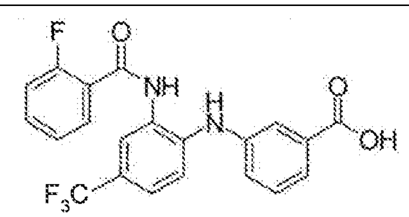 | |

The following Reference Examples are described to explain the present invention in more detail, and the present invention is not restricted to the following Reference Examples. Although the present invention is fully explained by way of the Reference Examples, the person skilled in the art understand that various alterations and modifications can naturally be made, Accordingly, these alterations and modifications are included in the present invention unless they depart from the scope of the present invention.

Reference Example 1

To a mixture of 1.0 g of ethyl 3~(5~cyano~1H~indol~1~yl)benzoate, 10 mL of methanol and 20 mL of THF was added 5.5 mL of 1M aqueous sodium hydroxide solution, followed by stirring at room temperature for 3 hours. 5.5 mL of a 1M aqueous hydrochloric acid and 50 mL of water were added to the reaction solution, and the precipitated solid was collected by filtration. The solid obtained was washed with ethanol-ether (1:2) to obtain 800 mg of 3~(5~cyano~1H-indol-~1-yl)benzoic acid as a white solid.
Compounds of Reference Examples 2 to 46 listed in Tables 14 to 17 were obtained in the same manner as in Reference Example 1.

Reference Example 47

60 mg of sodium hydride (55%, dispersion in paraffin liquid) was added under ice cooling to a mixture of 150 mg of 3-[3-(2-hydroxyethyl)-5-(trifluoromethyl)-1H-indol-1-yl]benzoic acid, 0.090 mL of methyl iodide and 2 mL of THF, followed by stirring under ice cooling for 3 hours and at room temperature overnight. 20 mL of water and 10 mL of 5% aqueous potassium hydrogen sulfate solution were added to the reaction solution, and the precipitated solid was collected by filtration. The solid obtained was washed with ether~hexane to obtain 110 mg of 3-[3-(2-methoxyethyl)-5-(trif1uoromethyl)-1H-indol-1-yl]benzoic acid as a pale yellow solid.

Reference Example 48

A mixture of 190 mg of 3-({2-[(cyciohexylcarbonyl)amino]-4-(trifluoromethyl)phenyl}amino)benzoic acid and 4 mL of acetic acid was stirred at 80˚C overnight. The solvent was evaporated under reduced pressure, and the residue was washed with ethanol to obtain 160 mg of 3-[2-cydohexyl-5-(trifluoromethyl)-1H-benzimidazol-1-yl]benzoic acid as a white solid.
Compounds of Reference Examples 49 to 53 listed in Tables 17 and 18 were obtained in the same manner as in Reference Example 48.

Reference Example 54

0.157 mL of a trifluoroacetic anhydride was added under ice cooling to a solution of 275 mg of 3-{[2-ammo-4-(trifluoromethyl)phenyl]amino} benzoic acid in 5 mL of THF, followed by stirring at room temperature for 30 minutes. The reaction solution was concentrated under reduced pressure, and 5 mL of acetic acid was added to the resulting residue, followed by stirring at 90˚C for 12 hours. After being cooled to room temperature, the reaction solution was concentrated under reduced pressure. The resulting residue was crystallized from a mixture of hexane and ethyl acetate to obtain 176 mg of 3-[2,5-bis(trifluoromethyl)-1H-benzimidazol-1-yl]benzoic acid as a pale yellow solid.

Reference Example 55

A solution of 163 mg of 3-[2-chloro-5-(trifluoromethyl)-1H-benzimidazol-1-yl]benzoic acid in 2 mL of DMF was added under ice cooling to a suspension of 163 mg of sodium ethoxide in 3 mL of DMF, followed by stirring at room temperature for 1 hour. 163 mg of sodium ethoxide was added to the reaction solution, followed by stirring at room temperature for 2 hours. 4.8 mL of 1 M aqueous hydrochloric acid was added under ice cooling to the reaction solution, and ethyl acetate and water were added thereto, followed by separation of the organic layer. The organic layer was washed with saturated brine and dried over anhydrous sodium sulfate, and then the solvent was evaporated under reduced pressure. The resulting residue was purified by silica gel column chromatography (eluent: chloroiorm-methanot=24:1), and the solid obtained was recrystallized from a mixture of hexane and ethyl acetate to obtain 202 mg of 3-[2-ethoxy-5-(trifluoromethyl)-1H-benzimidazol-1-yl]benzoic acid as a white solid.
A compound of Reference Example 56 listed in Table 18 was obtained in the same manner as in Reference Example 55.
The physicochemical data of compounds of Reference Examples 1 to 56 are shown in Tables 19 to 21.
[Table 14]

| Ex | Syn (Ex) | Str | Ex | Syn (Ex) | Str |
|---|---|---|---|---|---|
| 1 | 1 | 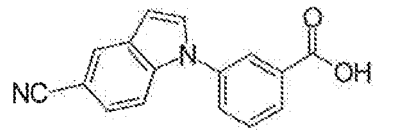 | 2 | 1 | 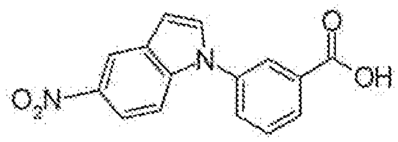 |
| 3 | 1 | 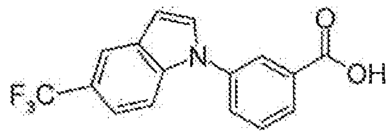 | 4 | 1 | 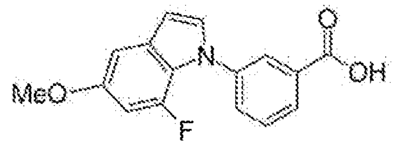 |
| 5 | 1 | 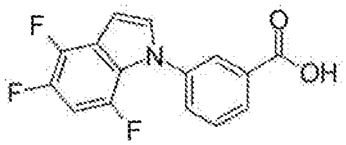 | 6 | 1 | 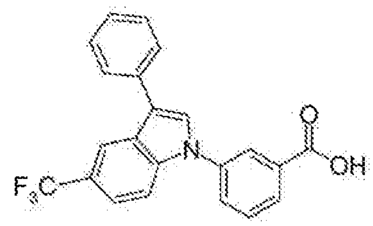 |
| 7 | 1 | 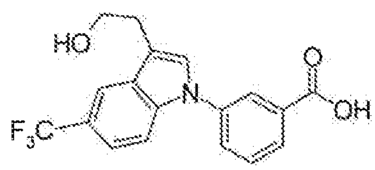 | 8 | 1 | 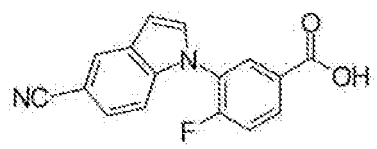 |
| 9 | 1 | 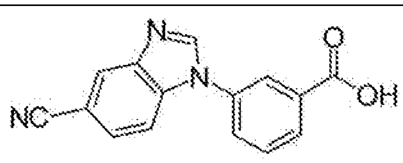 | 10 | 1 | 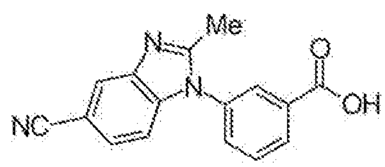 |
| 11 | 1 | 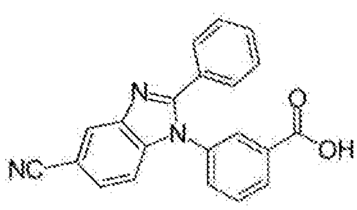 | 12 | 1 | 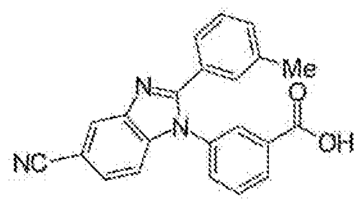 |
| 13 | 1 | 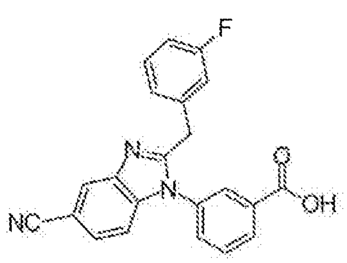 | 14 | 1 | 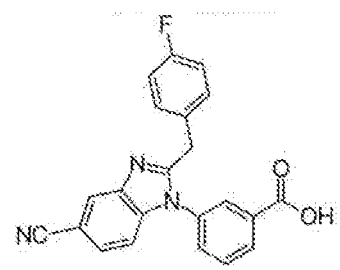 |

[Table 15]

| Ex | Syn (Ex) | Str | E x | Syn (Ex) | Str |
|---|---|---|---|---|---|
| 15 | 1 | 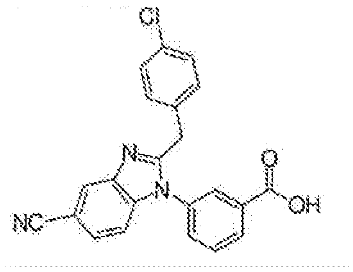 | 16 | 1 | 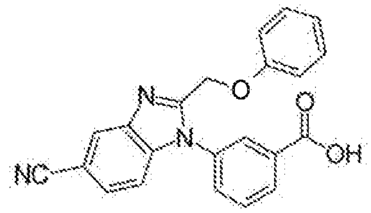 |
| 17 | 1 | 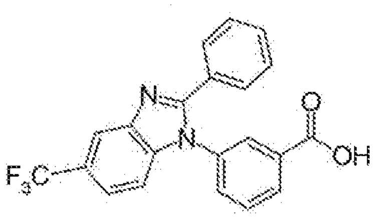 | 18 | 1 | 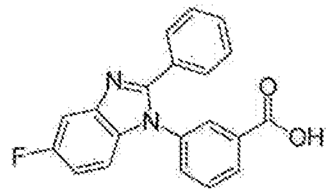 |
| 19 | 1 | 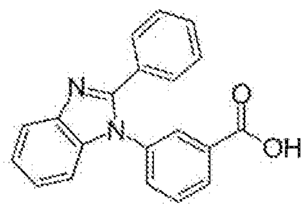 | 20 | 1 | 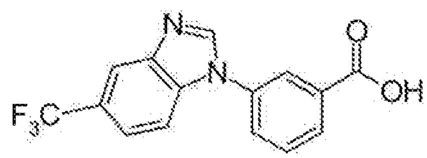 |
| 21 | 1 | 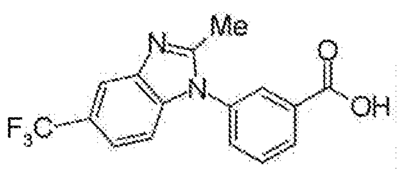 | 22 | 1 | 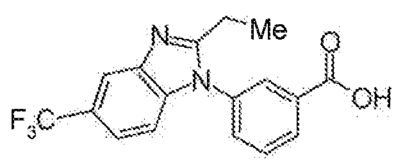 |
| 23 | 1 | 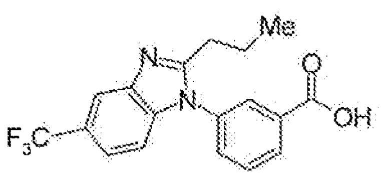 | 24 | 1 | 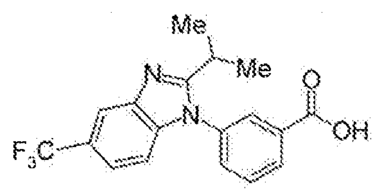 |
| 25 | 1 | 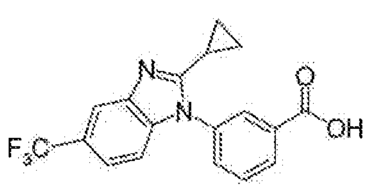 | 26 | 1 | 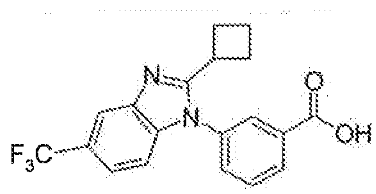 |
| 27 | 1 | 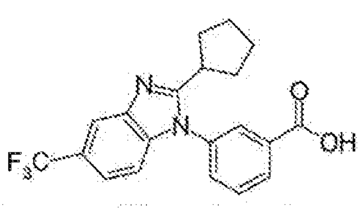 | 28 | 1 | 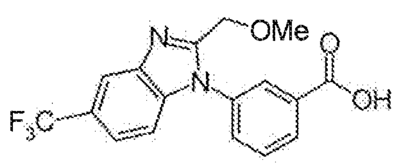 |

[Table 16]

| Ex | Syn (Ex) | Str | Ex | Syn (Ex) | Str |
|---|---|---|---|---|---|
| 29 | 1 | 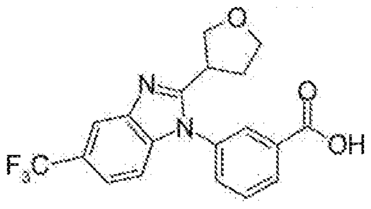 | 30 | 1 | 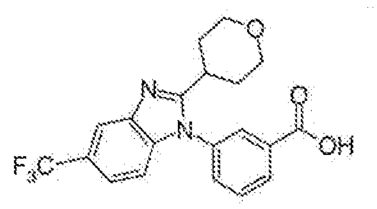 |
| 31 | 1 | 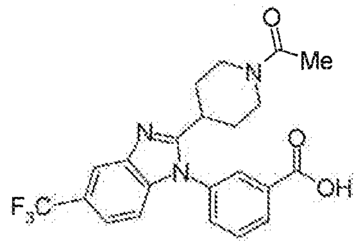 | 32 | 1 | 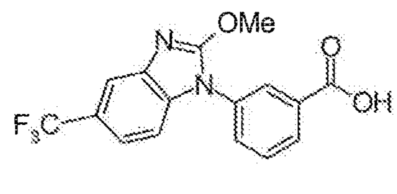 |
| 33 | 1 | 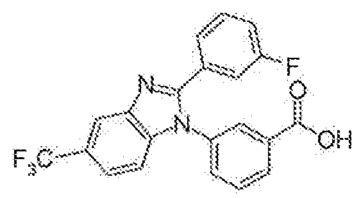 | 34 | 1 | 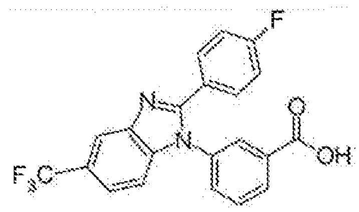 |
| 35 | 1 | 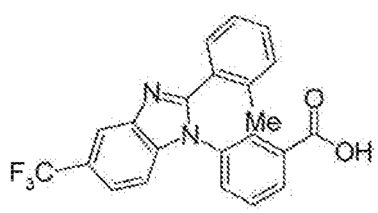 | 36 | 1 | 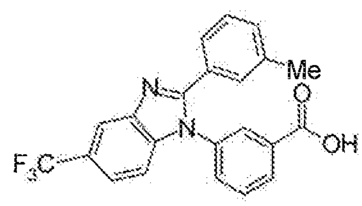 |
| 37 | 1 | 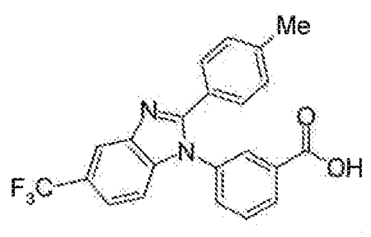 | 38 | 1 | 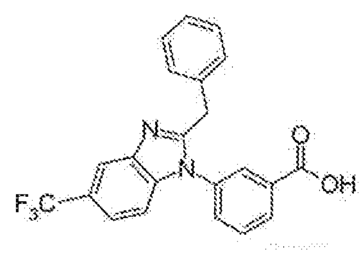 |
| 39 | 1 | 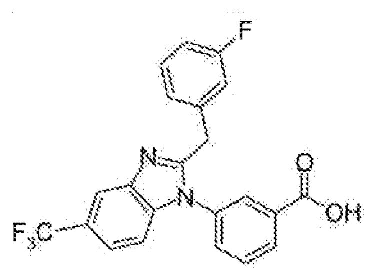 | 40 | 1 | 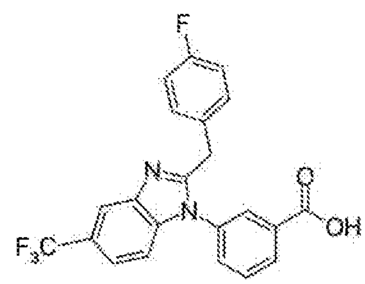 |

[Table 17]

| Ex | Syn (Ex) | Syn | Ex | Syn (Ex) | Str |
|---|---|---|---|---|---|
| 41 | 1 | 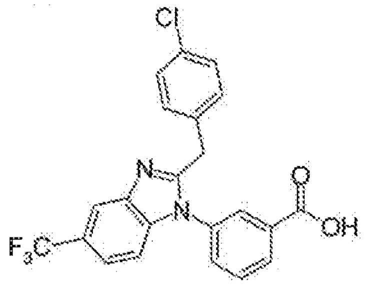 | 42 | 1 | 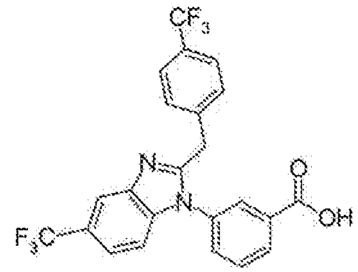 |
| 43 | 1 | 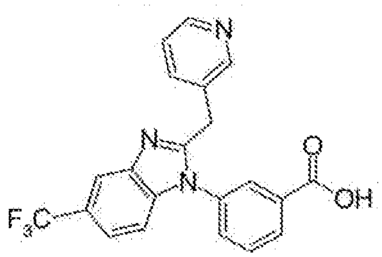 | 44 | 1 | 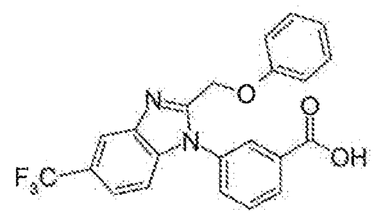 |
| 45 | 1 | 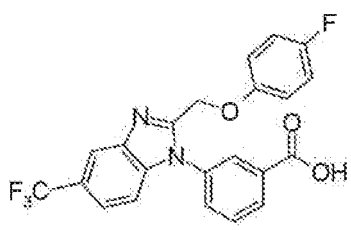 | 46 | 1 | 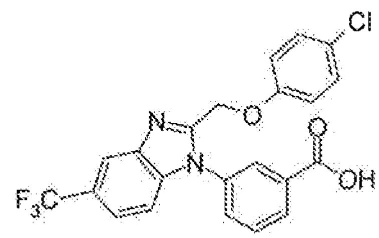 |
| 47 | 47 | 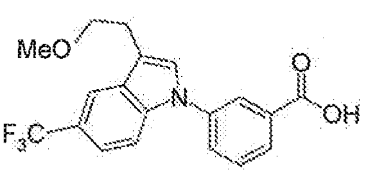 | 48 | 48 | 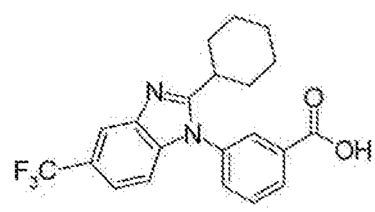 |
| 49 | 48 | 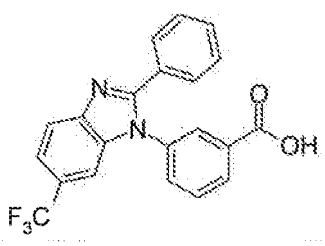 | 50 | 48 | 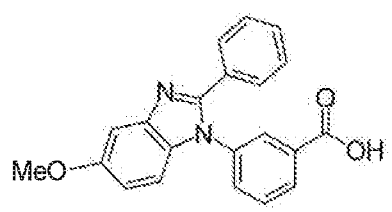 |
| 51 | 48 | 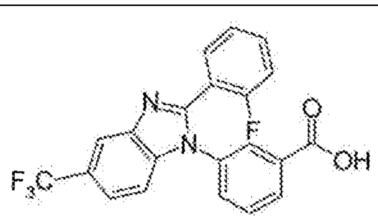 | 52 | 48 | 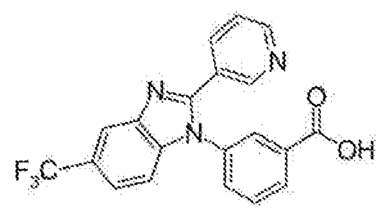 |

[Table 18]

| Ex | Syn (Ex) | Str | Ex | Syn (Ex) | Str |
|---|---|---|---|---|---|
| 53 | 48 | 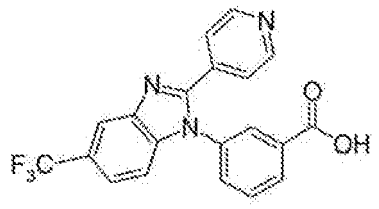 | 54 | 54 | 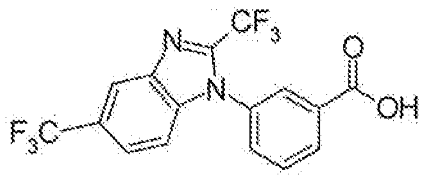 |
| 55 | 55 | 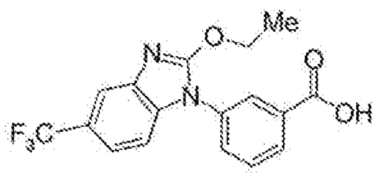 | 56 | 55 | 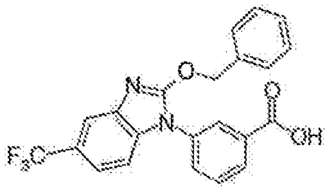 |

[Table 19]

| Ex | Data |
|---|---|
| 1 | NMR-DMSOd6: 6.90 (1H, d, J=3.3HZ), 7.58 (1H, dd, J=1.5, 8,6Hz), 7.68 (1H, d, J=8.6Hz), 7.75 (1H, t, J=7.8Hz), 7.88-7.93 (1H, m), 7.96 (1H, d, J=3.3Hz), 8.03 (1H, d, J=7.6Hz), 8.06 (1H, s), 8.24 (1H, d, J=0.8Hz), 13.35 (1H, s) FAB+: 261 |
| 2 | FAB-: 281 |
| 3 | FAB-: 304 |
| 4 | FAB+: 284 |
| 5 | FAB-: 290 |
| 6 | FAB-: 380 |
| 7 | ESI-: 348 |
| 8 | FAB+: 281 |
| 9 | ESI+: 264 |
| 10 | ESI+: 278 |
| 11 | ESI+: 340 |
| 12 | FAB+: 354 |
| 13 | ESI+ 372 |
| 14 | ESI+: 372 |
| 15 | ESI+: 388 |
| 16 | ESI+: 370 |
| 17 | NMR-DMSOd6: 7.37-7.49 (4H, m), 7.53-7.57 (2H, m), 7.62 (1H, d, J=8.6Hz), 7.70-7.77 (2H, m), 7.99 (1H, s), 8.10-8.14 (1H, m), 8,20 (1H, s), 13.37 (1H, s) FAB+: 383 |
| 18 | ESI+: 333 |
| 19 | ESI+: 315 |
| 20 | ESI+: 307 |
| 21 | NMR-DMSOd6: 2.48 (3H, s), 7.33 (1H, d, J=8.4Hz), 7.53 (1H, d, J=8.4Hz), 7.81 (1H, t, J=7.8Hz), 7.85-7.90 (1H, m), 8.01 (1H, s), 8.06 (1H, t, J=1.8Hz), 8.16 (1H, d, J=7.8Hz), 13.40 (1H, s) FAB+: 321 |

[Table 20]

| Ex | Data |
|---|---|
| 22 | FAB+: 335 |
| 23 | FAB+: 349 |
| 24 | FAB+: 349 |
| 25 | FAB+: 347 |
| 26 | ESI+: 361 |
| 27 | FAB+: 375 |
| 28 | FAB+:351 |
| 29 | FAB+: 377 |
| 30 | FAB+: 391 |
| 31 | FAB+: 432 |
| 32 | NMR-DMSOd6: 4.17 (3H, s), 7.35 (1H, d, J=8.4Hz), 7.48 (1H, d, J=8.4Hz), 7.76 (1H, t, J=8.0Hz), 7.85-7.87 (2H, m), 8.07-8.10 (2H, m), 13.36 (1H, s) FAB+: 337 |
| 33 | FAB+: 401 |
| 34 | FAB+: 401 |
| 35 | ESI+: 397 |
| 36 | FAB+: 397 |
| 37 | FAB+: 397 |
| 38 | NMR-DMSOd6: 4.21 (2H, s), 7.04 (2H, d, J = 7.3 Hz), 7.15-7.22 (3H, m), 7.30 (1H, d, J = 8.6 Hz), 7.54 (1H, d, J = 8.5 Hz), 7.73-7.76 (2H, m), 7.89 (1H, m), 8.08 (1H, s), 8.10-8.14 (1H, m), 13.33 (1H, s) ESI+: 397 |
| 39 | ESI+: 415 |
| 40 | ESI+: 415 |
| 41 | ESI+: 431 |
| 42 | ESI-: 463 |
| 43 | ESI+: 398 |
| 44 | NMR-DMSOd6: 5.33 (2H, s), 6.87-6.95 (3H, m), 7.21- 7.26 (2H, m), 7.42 (1H, d, J=8.7Hz), 7.63 (1H, dd, J=8.6, 1.5Hz), 7.76 (1H, t, J=7.8Hz), 7.89 (1H, dt, J=8.1, 1.6Hz), 8.12-8.14 (2H, m), 8.18 (1H, s), 13.34 (1H, s) FAB+: 413 |
| 45 | FAB+: 431 |
| 46 | FAB+: 447 |
| 47 | NMR-DMSOd6: 3.06 (2H, t, J=6.8Hz), 3.30 (3H, s), 3.67 (2H, t, J=6.8Hz), 7.52 (1H, d, J=8.8Hz), 7.68-7.78 (3H, m), 7.89 (1H, d, J=7.6Hz), 7.99 (1H, d, J=6.8Hz), 8.05 (1H, s), 8.09 (1H, s), 13.31 (1H, s) FAB-: 362 |

[Table 21]

| Ex | Data |
|---|---|
| 48 | NMR-DMSOd6: 1.09-1.31 (3H, m), 1.59-1.77 (5H, m), 1.83-1.91 (2H, m), 2.66-2.75 (1H, m), 725 (1H, d, J=8.4Hz), 7.52 (1H, d, J=8.4Hz), 7.82 (1H, t, J=7.8Hz), 7.87 (1H, d, J=7.8Hz), 8.03 (1H, s), 8.06 (1H, s), 8.18 (1H, d, J=7.8Hz), 13.31 (1H, s) FAB+: 389 |
| 49 | FAB+: 383 |

(continued)

| Ex | Data |
|---|---|
| 50 | FAB+: 345 |
| 51 | FAB+: 401 |
| 52 | FAB+: 384 |
| 53 | FAB+: 384 |
| 54 | ESI+: 375 |
| 55 | ESI+: 351 |
| 56 | ESI+: 413 |

Reference Example 57

To a solution of 6 mg of isovaleryl chloride in 0.2 mL of THF were added a solution of 13 mg of ethyl 3-[2-amino-4-(trifluoromethyl)phenyl]aminobenzoate and 0.02 mL of pyridine in 0.3 mL of THF, followed by stirring at room temperature overnight. Water and chloroform were added thereto, the organic layer was separated, and the solvent was evaporated under reduced pressure. 0.5 mL of acetic acid was added to the resulting residue, followed by stirring at 90˚C overnight and then the solvent was evaporated under reduced pressure. The resulting residue was dissolved in 0.2 mL of methanol, 0.2 mL of THF, 0.1 mL of a 5.M aqueous sodium hydroxide solution, followed by stirring at 60˚C overnight. After being cooled to room temperature, neutralizing with 1M aqueous hydrochloric acid, the solvent was evaporated under reduced pressure. The resulting residue was fractionated and purified by HPLC to obtain 4.1 mg of 3-[2-isobutyl-5-(trifluoromethyl)-1H-benzimidazol-1-yl]benzoic acid.

HPLC conditions used for purification

Column: SunFire (trademark) (particle size: 5 μm, inner diameter: 19 mm, and length: 100 mm)

Mobile phase: A sol, methanol, and B sol, 0.1 % aqueous formic acid solution

Table 22

| Time (min) | A Sol (%) | B Sol (%) |
|---|---|---|
| 0-1 | 10 | 90 |
| 1-9 | 10→95 | 90→5 |
| 9-12 | 95 | 5 |

Flow rate: 25 mL/min

Column temperature: 20˚C

Injection volume: 800 μL

The conditions of HPLC performed for the determination of RT are shown below.

Column: Wakosil-II 5C18AR (trademark) (particle size: 5 μm, inner diameter: 2.0 mm, and length: 30 mm)

Mobile phase: A sol, 5 mM aqueous trifluoroacetic acid solution, and B sol, methanol

[Table 23]

| Time (min) | ASol (%) | BSol (%) |
|---|---|---|
| 0-4 | 90→0 | 10→100 |
| 4-4.5 | 0 | 1 0 0 |

Flow rate: 1.2mL/min

Detection wavelength: 254 nm

Column temperature: 35.0˚C

Injection volume: 5 μL

The compounds of Reference Examples 58 to 60 listed in Table 24 were obtained in the same manner as in Reference

Example 57,

[Table 24]

| Ex | Str | RT | ESI+ |
|---|---|---|---|
| 57 | 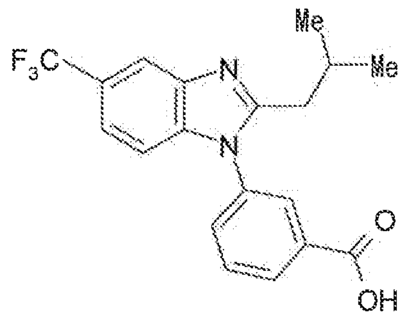 | 2.82 | 363 |
| 58 | 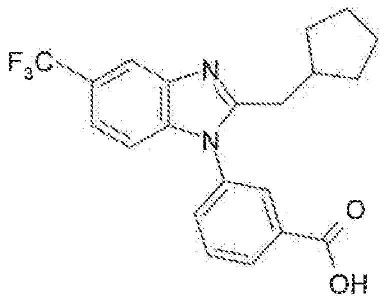 | 3.04 | 389 |
| 59 | 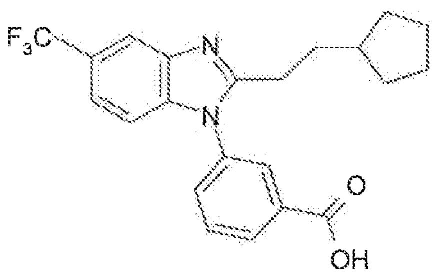 | 3.21 | 403 |
| 60 | 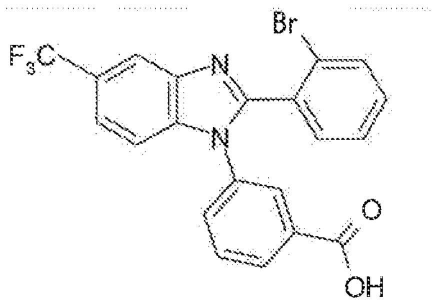 | 2.98 | 461 |

Example

Based on the following Examples, the present invention relating to a screening method for an agent for treating 17βHSD type 5-related diseases and/or an agent for treating 17βHSD type 5-reiated cancer such as prostate cancer will be described in more detail; however, the present invention is not limited to the Examples.

A mouse to which CWR22R, a cell strain derived from human prostate cancer, had been transplanted was prepared to evaluate the testosterone production suppressive activities of the Reference Example compounds in the tumor.

Example 1

Testosterone Production Suppression in the Tumor of a CWR22R Tumor-bearing Mouse

As the Matrigel described below, BD Matrigel (trademark) phenol red-free 356237 (manufactured by Becton, Dickinson and Company) was used.

A CWR22R tumor was excised from a CWR22R subcutaneous tumor-bearing nude mouse, The excised tumor was segmented to become tumor specimens with size of about 5 mm per specimen. The tumor specimens were washed with a 20% serum-containing RPM11640 culture medium twice and then stirred for about 20 minutes in a 0.1 % protease and 20% serum-containing RPMI1640 culture medium. Supernatant was recovered to recover cells through centrifugation. These procedures were repeated as necessary, recovered cells were collected, and thereby the cell solution was prepared. After the cell number was counted, the cell solution was adjusted to $2\times10^7$ cclls/mL. The same amount of the cell solution and Matrigel were mixed and adjusted to the final value of $1\times10^7$ cells/mL. 0.1 mL of the cell Matrigel mixture prepared in the above-described manner was subcutaneously injected to one site of the back of a male nude mouse with testectomy for the transplant. After the transplant, at a time point when the tumor volume reached 100 $mm^3$ or more, grouping was performed based on the tumor volume to conduct the following experiments.

Test substances were orally administered to the CWR22R subcutaneous tumor-bearing mouse. After an hour, that is, an hour before dissection, androstenedione was administered into the tumor to yield a level of 1 ng/100$mm^3$ in the tumor. The composition of the liquid for administering androstencdione included 0.000075% DMF/0.00005% polysorbate 80/9.999875% saHne/90% Matrigel. Tumor excision was performed through the dissection, and the tumor was weighed and then frozen for preservation. For the purpose of measuring the background value, the tumor of the mouse to which only the solvent for administering test substances had been orally administered was excised. Subsequently, under the ice cooling, the liquid for administering androstenedione was added thereto, and the resultant was subjected to quick-freezing for preservation.

To the tumor sample preserved through freezing was added a 0.2 M Na-K phosphate buffer (pH 7.4), and thereby a tumor homogenate was prepared under ice cooling by using a homogenizer (polytron). Tert-butyl methyl ether with a 6-fold capacity was added to the tumor homogenate, followed by centrifugation (3000 rpm, 5 min), and the supernatant was transferred to another test tube. While the test tube was maintained at 40˚C in a heat block, the solvent was evaporated by nitrogen gas. 2% BSA-containing 10 mM Tris-Cl buffer (pH 7.4) was added to the test tube and mixed to obtain an extract, and the testosterone concentration was measured by using DELFIA (trademark) Testosterone Reagents R050-201 (manufactured by PerkinElmer, Inc.).

The testosterone production suppression rates (%) of some Reference Example compounds in the tumor of CWR22R tumor-bearing mouse are shown in Table 25. Ex represents a Reference Example number, Inh represents the testosterone production suppression rates upon administration of 10 mg/kg of the Reference Example compounds.

The screening method above can also evaluate the estrogen production suppression rate by using MCF-7 cell or the like instead of CWR22R, a tumor cell derived from prostate cancer, further, by using estrone or the like instead of androstenedione.

From the above, results, it became clear that the screening method of the present invention is valuable as a screening method for compounds which are useful for treating 17βHSD type 5-related diseases, for example, prostate cancer, breast cancer or the like.

[Table 25]

| Ex | Inh (%) (10mg/kg) |
|---|---|
| 17 | 75 |
| 20 | 89 |
| 21 | 97 |
| 23 | 96 |
| 36 | 45 |
| 38 | 66 |
| 39 | 31 |
| 40 | 49 |
| 41 | 45 |
| 45 | 94 |

(continued)

| Ex | Inh (%) (10mg/kg) |
|---|---|
| 47 | 33 |
| 54 | 99 |

Industrial Applicability

According to the screening method of the present invention, it is possible to efficiently evaluate candidate substances which can be agents for treating 17βHSD type 5-related diseases and/or agents for treating prostate cancer. Also, it is possible to obtain test results with excellent reproducibility and small irregularity by dissolving a steroid, which is a biosynthetic material for a hormone, in a gel-type substances, and topically administering it into the tumor.

## Claims

**1.** A screening method for a compound which is useful for treating 17βHSD type 5-related diseases, which comprises manifesting a tumor by transplanting tumor cells to an animal to be tested and measuring the level of a hormone produced in the tumor.

**2.** The screening method according to claim 1, which comprises:

1) a process of manifesting the tumor by transplanting tumor cells to an immunodeficient mouse;
2) a process of administering a test substance to the immunodeficient mouse;
3) a process of topically administering a steroid which is a biosynthetic material for a hormone into the tumor;
4) a process of measuring the hormone concentration in the tumor of the immunodeficient mouse and calculating the inhibition rate in conversion of the steroid which is a biosynthetic material for a hormone to the hormone; and
5) a process of selecting a substance which suppresses the hormone production in the tumor.

**3.** The screening method according to claim 2, wherein a cell solution is diluted using a gel-type substance selected from collagen, Matrigel, silicon glue and gelatin, for the transplantation, in the process of manifesting the tumor by transplanting tumor cells to an immunodeficient mouse.

**4.** The screening method according to claim 3, wherein the steroid is dissolved or suspended into a gel-type substance selected from collagen, Matrigel, silicon glue and gelatin, for the administration, in the process of topically administering a steroid which is a biosynthetic material for a hormone into the tumor.

**5.** The screening method according to any one of claims 1 to 4, wherein the hormone is testosterone.

**6.** A compound selected by the screening method according to any one of claims 1 to 5 or a salt thereof.

**7.** A pharmaceutical which comprises the compound according to claim 6 or a salt thereof as an active ingredient.

**8.** The pharmaceutical according to claim 7, which is an agent for treating 17βHSD type 5-related diseases.

**INTERNATIONAL SEARCH REPORT**

International application No.
PCT/JP2010/050937

A. CLASSIFICATION OF SUBJECT MATTER
*G01N33/50*(2006.01)i, *A61K45/00*(2006.01)i, *A61P31/00*(2006.01)i, *G01N33/15*(2006.01)i

According to International Patent Classification (IPC) or to both national classification and IPC

B. FIELDS SEARCHED

Minimum documentation searched (classification system followed by classification symbols)
G01N33/48-G01N33/98, G01N33/15

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CA(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| A | WO 2007/100066 A1 (Astellas Pharma Inc.), 07 September 2007 (07.09.2007), & US 2009/0181960 A & EP 1990335 A1 & CA 2644809 A & KR 10-2008-0114711 A & CN 101395134 A | 1-5 |
| A | WO 2008/149521 A1 (Immuno-Biological Laboratories Co., Ltd.), 11 December 2008 (11.12.2008), paragraph [0025] (Family: none) | 1-5 |
| A | JP 8-512292 A (The United States of America), 24 December 1996 (24.12.1996), & JP 8-512292 A & US 5698413 A & EP 697892 A & WO 1994/025074 A1 & DE 69432861 T | 1-5 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 April, 2010 (08.04.10) | 20 April, 2010 (20.04.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2010/050937

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 6-8
because they relate to parts of the international application that do not comply with the prescribed requirements to such an extent that no meaningful international search can be carried out, specifically:
See extra sheet.

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.
PCT/JP2010/050937

Continuation of Box No.II-2 of continuation of first sheet(2)

The compound or a salt thereof described in claim 6, the pharmaceutical preparation described in claim 7 that refers to claim 6, and the pharmaceutical preparation described in claim 8 that refers to claim 7 are specified by a screening method, i.e., these products are specified as being "selected by the screening method described in any one of claims 1-5". Therefore, these products include all of the compounds and the pharmaceutical preparations obtained by the screening method.

However, even though the common technical knowledge at the time of filing the present application is taken into consideration, it is quite unclear as to what types of compounds or pharmaceutical preparations are specifically included or what types of compounds or pharmaceutical preparations are specifically excluded. Therefore, claims 6-8 are significantly unclear, and do not comply with the requirement of clarity under PCT Article 6.

Such being the case, a meaningful search cannot be carried out on the inventions described in claims 6-8.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 99046279 A
- WO 2004064735 A
- WO 2003057670 A
- US 20070105862 A
- WO 2001007020 A
- WO 2006041874 A
- EP 563001 A
- EP 616807 A
- US 5554630 A
- WO 96033194 A
- WO 98017651 A
- WO 98034923 A
- JP 7133224 A
- WO 97033872 A
- JP 2002193947 A
- WO 2008006790 A


### Non-patent literature cited in the description


- *The New England Journal of Medicine,* 2003, vol. 349, 2387-2398
- *Frontier in Neuroendocrinology,* 2001, vol. 22, 185-212
- *The Journal of Urology,* 1999, vol. 161, 332-337
- *The Journal of Clinical Endocrinology and Metabolism,* 1995, vol. 80, 1066-1071
- *Clinical Cancer Research,* 2004, vol. 10, 440-448
- *Steroids,* 2004, vol. 69, 795-801
- *Cancer Research,* 2006, vol. 66, 2815-2825
- *Nature Genetic,* 1994, vol. 7, 34-39
- *Endocrine Reviews,* 2003, vol. 24, 152-182
- *The Journal of Biological Chemistry,* 2002, vol. 277 (27), 24799-24808
- *Carcinogenesis,* 2004, vol. 25 (11), 2177-2181
- *Cancer Research,* 2004, vol. 64, 1802-1810
- *Molecular and Cellular Endocrinology,* 2006, vol. 248, 233-235
- *The Journal of Medicinal Chemistry,* 1998, vol. 41 (27), 5457-5465
- *Bioorganic and Medicinal Chemistry,* 2004, vol. 12 (15), 4009-4015
- *Bioorganic and Medicinal Chemistry,* 2005, vol. 13 (24), 6598-6608
- *Cancer Research,* 1996, vol. 56, 3042-3046
- *Journal of Steroid Biochemistry,* 1988, vol. 30, 311-314
- *Prog. Med.,* 1985, vol. 5, 2157-2161
- pharmaceutical Research and Development. Drug Design. Hirokawa Publishing Company, 1990, vol. 7, 163-198
- Protective Groups in Organic Synthesis. 1999
- Courses in Experimental Chemistry. Maruzen, 1992, vol. 20
- Courses in Experimental Chemistry. Maruzen, 2005, vol. 14
- *Compendium of Organic Synthetic Methods,* vol. 1-3
- Courses in Experimental Chemistry. Maruzen, 1992, vol. 26
- *Synthesis,* 1982, vol. 10, 876-878
- Courses in Experimental Chemistry. Maruzen, 1992, vol. 22
- Courses in Experimental Chemistry. Maruzen, 2005, vol. 16
- *Compendium of Organic Synthetic Methods*
- *Journal of the American Chemical Society,* 2001, vol. 123, 7727-7729
- **Maniatis, T. et al.** Molecular Cloning-A Laboratory Manual. Cold Spring Harbor Laboratory, 1992
- *Molecular Endocrinology,* 1997, vol. 11 (13), 1971-1984
- **Trevor M. Penning et al.** *Biochem. J.,* 2000, vol. 351, 67-77